(19) Europäisches Patentamt / European Patent Office / Office européen des brevets

(11) **EP 3 679 891 B1**

(12) **EUROPEAN PATENT SPECIFICATION**

(45) Date of publication and mention of the grant of the patent:
**01.01.2025 Bulletin 2025/01**

(21) Application number: **18850024.3**

(22) Date of filing: **04.09.2018**

(51) International Patent Classification (IPC):
***A61F 2/24*** *(2006.01)*

(52) Cooperative Patent Classification (CPC):
**A61F 2/2418; A61F 2/2436**

(86) International application number:
**PCT/CN2018/103973**

(87) International publication number:
**WO 2019/042469 (07.03.2019 Gazette 2019/10)**

(54) **STENT DEVICE HAVING SKIRT FOR PERIPHERAL LEAKAGE PREVENTION AND PROCESSING METHOD THEREOF, SKIRT FOLDING METHOD, AND HEART VALVE**

STENTVORRICHTUNG MIT SCHÜRZE FÜR PERIPHERE LECKVERHINDERUNG UND VERFAHREN ZU IHRER VERARBEITUNG, SCHÜRZENFALTVERFAHREN UND HERZKLAPPE

DISPOSITIF DE STENT COMPORTANT UNE JUPE DESTINÉE À LA PRÉVENTION DE FUITE PÉRIPHÉRIQUE ET MÉTHODE DE TRAITEMENT CORRESPONDANTE, MÉTHODE DE PLIAGE DE JUPE ET VALVE CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2017 CN 201710786392**
**28.10.2017 CN 201711031064**

(43) Date of publication of application:
**15.07.2020 Bulletin 2020/29**

(73) Proprietor: **Venus MedTech (HangZhou) Inc.**
**Binjiang District**
**Hangzhou**
**Zhejiang 310052 (CN)**

(72) Inventors:
• **KUANG, Dajun**
**Hangzhou**
**Zhejiang 310052 (CN)**
• **YU, Jincheng**
**Hangzhou**
**Zhejiang 310052 (CN)**
• **LIM, Housen**
**455234 (SG)**

(74) Representative: **Novagraaf Group**
**Chemin de l'Echo 3**
**1213 Onex / Geneva (CH)**

(56) References cited:
WO-A1-2015/152980    WO-A2-2009/044082
CN-A- 103 705 315    CN-A- 103 889 472
CN-A- 105 188 609    US-A1- 2016 317 305
US-A1- 2017 189 174

## Description

## TECHNICAL FIELD

The present application relates to the technical field of medical apparatuses, and in particular, to an artificial stent apparatus for a heart, or a blood vessel.

## BACKGROUND

Since the 1970s, the artificial heart valve replacement, as an effective treatment for end-stage heart valve disease, has saved millions of patients' lives. The artificial heart valve replacement is a revolutionary breakthrough in heart valve treatment technology, and has broad prospects.

Perivalvular leakage (PPL) is a serious and unique postoperative complication of valve replacement and is one of the common reasons which cause reoperations. The reason why PPL occurs is mainly related to the pathological changes of the annulus tissue such as degenerative changes, rheumatic or senile calcification, acute infective endocarditis invading the annulus or perivalvular abscess, mismatching of the artificial valve and the annulus in size, and the prosthetic valve endocarditis.

Peripheral leakage occurs not only when implanting artificial stent in the heart, but also in blood vessels or other body channels. For ease of description, perivalvular leakage is mainly taken as an example here.

The perivalvular leakage usually has the following adverse effects:(1) severe hemolysis, anemia, and progressively increased hemoglobinuria; (2) abnormal changes in hemodynamics, failure of heart function or even heart failure caused by large orifices for perivalvular leakage, with poor symptoms and signs improvement after conservative treatments; (3) infective endocarditis concurrent even with a small orifice for perivalvular leakage; and (4) bioprosthetic valve failure concurrent with a perivalvular leakage.

At present, some researches have been done on the prevention of perivalvular leakage, at home and abroad. However, the perivalvular leakage cannot be decreased to a rate below 5% during transcatheter replacement yet. Therefore, it is desired for further researches on how to reduce the perivalvular leakage.

At present, the main method of transcatheter bioprosthetic valve implantation for the treatment of heart valve disease is to compress an artificial heart valve into a delivery device, which delivers the valve to a lesion site of the heart through blood vessels, and then release the artificial heart valve to replace the diseased native valve. For example, WO2009044082A2 discloses a device including an implant which has a tubular endoprosthesis with a radially deployable framework. The implant includes a flexible interposition member mounted movably on the endoprosthesis so as to be interposed between the outer surface and a blood circulation conduit. The device includes a deployment tool which can adopt a configuration for insertion of the implant and a configuration for release of the implant. In the configuration for insertion of the implant, the flexible member includes at least one free part held axially away from the outer surface in order to minimize the radial dimension of the implant. The free part is at least partly displaceable against the outer surface when the tool moves from its configuration for insertion to its configuration for release. Typical heart valves include balloon expandable valves or self-expanding artificial heart valves. The self-expanding artificial heart valve generally includes a meshed stent made of a shape memory metal material, and a valve which opens unidirectionally sewn in the stent. During implantation, the stent expands itself and fits the diseased annulus as much as possible. Although the perivalvular leakage can be reduced to a certain extent, the perivalvular leakage and peripheral back flow will still occur more or less to some patients who have blood vessels with irregular inner walls due to autocalcification. The stent of the balloon expandable valve is usually made of medical stainless steel. During implantation, the stainless steel stent is expanded by the balloon and fits the diseased annulus as much as possible.

The typical balloon expandable valve or self-expanding valve expands the inner wall of the blood vessel only by the radial support force of the metal stent. For patients who have blood vessels with regular inner walls, appropriate stent may be chosen to substantially fit the inner walls of the blood vessels by valve selection, which can reduce perivalvular leakage to a certain degree. However, for patients who have blood vessels with irregular inner walls caused by calcification, perivalvular leakage will occur more or less. A large number of clinical studies indicate that it is difficult to reduce the perivalvular leakage to a rate below 5%, and the risk of perivalvular leakage still exists. At present, once a patient suffers from perivalvular leakage, it can only be solved by measures such as repair and valve replacement, which result in a high risk for surgery and thus to the patient's life.

Although there have some typical solutions, all of these solutions have certain shortcomings. For example, paravalvular leak may be prevented by providing an annular pocket around the periphery of a stent, which would be filled with back-flow blood. However, it is likely to generate thrombus in the ring-shaped pocket, and there is a risk that the thrombus may fall off.

There are also some solutions which block perivalvular leakage by forming an annular protrusion protruding radially with water-absorbing expansion material on the outer periphery of the stent, while there is also a risk that the water-absorbing expansion material may fall off, which results in potential danger.

There are also some solutions provided with a flexible film at an inflow end of the stent, wherein the flexible film is located at an axial side of the stent before being released, and rolled up or folded up near the inflow end of the released stent to form an annular protrusion protruding radially so as to block perivalvular leakage.

However, as the flexible film is provided at the axial side of the stent before being released, which make the entire length of the resulted stent apparatus too long, which results in higher requirements for a delivery system. More importantly, in order to achieve the rolling or folding of the flexible film, an additional dragging mechanism must be provided, which is controlled by an operator and thus increases the complexity and difficulty of control. Alternatively, a metal member may be provided, which, based on the characteristic of preset shape thereof, drives the flexible film to roll up or fold up after being released in the human body. However, the metal member makes the structure of the stent more complicated, and brings additional risks. Furthermore, under the influence of the space outside of the stent, it is difficult to pull the flexible film to the desired position. Therefore, the effects are not satisfied.

## SUMMARY

The invention is defined in the appended claims and provides a stent apparatus with skirt for preventing peripheral leakage, which forms a peripheral leakage occluder by a self-stacked flexible skirt around an outer periphery of a stent after being released. More importantly, the flexible skirt is driven to stack by a deformed stent of the stent apparatus itself during release, that is, the skirt operates in cooperation with the deforming of the stent, without requirements for additional control and a metal part. The stent apparatus of the invention is able to seal the outer periphery of the stent flexibly, with safe operation and convenient processing.

A stent apparatus with skirt for preventing peripheral leakage, comprising a stent, wherein, the stent apparatus is further provided with at least one flexible skirt, which has:

an unfolded configuration, in which the skirt is axially unfolded and surrounding an outer periphery of the stent before being released;

a stacked configuration, in which the skirt is folded and stacked in an axial direction of the stent after being released and forms an annular peripheral leakage occluder;

wherein, the stent apparatus is further provided with a pulling string for driving the skirt to transform into the stacked configuration, the pulling string comprises a driving portion and a force exerting portion, wherein the driving portion operates in cooperation with the stent during release, the force exerting portion is connected with the skirt and is pulled by the driving portion during release of the stent to form an axial displacement thereof relative to the stent so as to pull the skirt to be stacked. The stent itself of the invention may be any of various known stents, and the main improvement of the invention is the inventive skirt located around the outer periphery of the stent. Individual configurations of the skirt are configured to adapt to a released stent and an unreleased stent, respectively. The stent takes a radially compressed configuration, after being loaded into a delivery system, i.e. a configuration before being released. Correspondingly, the skirt takes the unfolded configuration. In order to facilitate the travel of the stent in the human body, the stent should be compressed as much as possible. Correspondingly, the skirt is unfolded in the axial direction. The invention also differs from the prior art in that the skirt surrounds around the outer periphery of the stent before being released, at least with most part of the skirt surrounding around the outer periphery of the stent, rather than being located at an axial side of the stent. Since the skirt is thin and has a good flexibility, it is able to conform the outer periphery of the stent very well.

After being delivered into the human body with the delivery system, the stent starts to be released by withdrawing a sheath of the delivery system and gradually expands radially. After the release is completed, the shape of the stent remains relatively stable and fixed. During the release of the stent, the radial expansion of the stent is accompanied by changes in the circumferential direction, i.e., perimeter increasing.

The stent after being released is stably placed at the corresponding lesion site. Correspondingly, the skirt takes the stacked configuration. The radial expansion of the stent will drive the skirt and initiate axially folding and stacking in the axial direction, and to be thickened around the outer periphery of the stent, so as to form the annular peripheral leakage occluder.

Being axially unfolded of the skirt in the unfolded configuration is to minimize the radial dimension of the stent. In addition, the skirt will be axially unfolded by the sheath during load. The term axially unfolded described in the present invention should not be strictly limited to the meaning that the skirt "must" be axially unfolded and straighten, but should be interpreted as the skirt "may" be axially unfolded due to the structure of the skirt and the position or connection relationship between the skirt and the stent.

There is no strict limit to the specific stacked structure of the stent during axially folding and stacking, provided that axially folding the skirt will at least cause a thickening of the skirt in the radial direction for sealing the peripheral leakage.

Many alternative or preferred arrangements are further provided only for additional or preferred implementation, without any limitation to the above general solution. Without conflict in technique or logic, the arrangement may be combined with the above general solution alone or multiple arrangements may be combined with each other. Optionally, the skirt transitions into the stacked configuration under support of radial deforming of the stent during release.

As one of the manners to drive the skirt to transform into the stacked configuration, the deforming of the stent being released directly acts on the skirt and supports the same. The stent is not released instantaneously, but works in progress. With the stent gradually exposing from the delivery system, an end of the stent which is released first expands radially first and forms a flared configuration, and the deformation gradually transfers to the other end of the stent which is released later,. During this process, the gradually formed flared section drives the skirt to expand and guides the skirt to be folded and stacked axially. In addition, in the case of the end of the stent which is released first facing opposite to a direction of the blood flow, the impact on the skirt by the blood flow may also facilitate the skirt to be folded axially.

Optionally, the skirt is made of a flexible material and constrains around the outer periphery of the stent in a released configuration.

The skirt made of an elastic material and constraining around the outer periphery of the stent after being released is easy to be folded axially and maintained stably in the stacked configuration after being released, avoiding self-expanding axially of the skirt and failure to block the peripheral leakage.

The stent of the present invention may be a known stent, for example, with a generally cylindrical shape, and being processed by braiding or cutting. It has a meshed structure with a plurality of grids arranged regularly or combined partially with other structures having different shapes.

The stent may be applied in the aorta, aortic valve, pulmonary artery, pulmonary valve, mitral valve, tricuspid valve, occluding device, general blood vessel or the like. The shape of the stent and the presence of the coverage membrane may be adaptable based on the prior art as required.

Preferably, one section of the stent in the axial direction is configured as a flared driving structure that is configured to drive the skirt to be folded and stacked.

The stent may take a flared configuration during the release. In order to further ensure that the skirt can be folded and stacked in the axial direction, the stent itself may also form a flared driving structure at a section thereof corresponding to the skirt. In addition, the guidance of the flared structure facilitates to maintain the skirts in the stacked configuration after the stent is completely released without loosening in the axial direction, so as to ensure the function of the peripheral leakage occluder.

Preferably, the flared driving structure is located at a middle portion of the stent in the axial direction, or adjacent to axial ends of the stent.

Preferably, the flared driving structure is adjacent to an axial end of the stent, and is flared at the corresponding end.

Preferably, the flared driving structure is adjacent to the blood inflow end of the stent, and the closer to the blood inflow end, the wider opening the flared driving structure has. An appropriately orientated flared driving structure may operate in cooperation with the blood flow to push the skirts to axially stack A blood flow from the wider opening to the narrow opening has a function in compliance with the guidance of the flared driving structure. It is further preferred that the wider opening side of the flared driving structure is released first, and the narrow opening side is released later, which works on the skirt in cooperation with the flared structure of the stent during the release, so as to guide the skirt to be stacked axially and maintain the skirt in the stacked configuration.

Preferably, the flared driving structure extends to the distal end of the stent which is to be released first.

The flared driving structure extending to the distal end of the stent which is to be released first has the following advantages, when the stent starts to be released, the flared structure thereof presents clearly, and in addition, the axially limiting to the peripheral leakage occluder may be maintained steadily.

Optionally, the generatrix of the flared driving structure is a straight line, a smooth curved line, or a combination of a straight line and a curved line.

The shape of the generatrix determines the extension of the flared mouth and is not strictly limited in the present invention. The generatrix may be a single straight line or smooth curved line, or a combination of a straight line and a curved line, provided that a generally flared structure is formed and the flared driving structure should at least function at the distal end of the skirt to be released first.

Preferably, neither of two ends of the skirt in the unfolded configuration in the axial direction of the stent extends beyond the corresponding side of the stent.

When the stent is being released, the distal end which is to be released first will turn outwards. If the skirt in the unfolded configuration covers the side with the tips, the distal end of the stent to be released first may snag the inner side of the skirt or even pierce the skirt, which would prevent the skirt from being folded axially. Therefore, it is preferred that at least a part of the stent is released at the earliest and then works on the skirt so as to automatically guide the skirt to be stacked.

Preferably, one axial side edge of the stent has a sharp angled structure, and before the stent is released, the corresponding side of the skirt does not extend axially beyond the tips of the sharp angled structure.

The typical stents are generally in meshed structures, with grid end nodes or sharp tips at the distal end to be released first, which are likely to snag on the skirt during release. Before the stent is released, the corresponding side of the skirt may be unfolded in a single layer, rolled inwards/outwards, folded or stacked. In any event, it is preferred that the axial edge of the skirt does not extend beyond the tips of the sharp angled structure at the corresponding side. Preferably, before the stent is released, at least a portion of the distal end of the stent to be released first is exposed outside the skirt, and the exposed portion is released before the skirt.

Preferably, a section of the stent in the axial direction is a flared driving structure that drives the skirt to be folded and stacked, and at least a portion of the flared driving structure is configured as said exposed portion.

It is apparent to have a coordinative effect by releasing the stent before the skirt in combination with said flared driving structure.

In order to facilitate the stack of the skirt, at least to prevent the skirt from being "snagged" during the release of the stent, it is further preferred that an axial end of the stent has a smoothly curved edge. Due to such configuration, the corresponding side of the skirt may extend axially beyond the stent before the stent is released.

For example, the edge of the distal end of the stent to be released first is formed by a plurality of curved segments connected one another, and the skirt extends axially beyond the distal end of the stent to be released first. The curved structure would not pierce or snag the skirt, so it is allowable for the skirt to extend axially beyond the distal end of the stent to be released first in such a way that a peripheral leakage with higher profile and larger volume may be formed by the longer skirt, thereby improving the sealing effect.

Preferably, before the stent is released, the skirt in the unfolded configuration has a folded structure in a circumferential direction.

Since the stent before being released has a low profile, the skirt may be attached to the outer periphery of the stent relatively flat due to the folded structure in the circumferential direction. After the stent is released, the circumferentially folded structure is unfolded due to the radial expansion of the stent.

Preferably, the stent is provided with a valve and/or an inner coverage membrane..

As further preferred, the skirt and the valve and/or the inner coverage membrane are made of same material.

The stent may be provided with a valve and/or a coverage membrane according to its particular applications, i.e., the lesion sites. It is required for the skirt to be able to be folded and stacked, so the skirt is made of flexible material and at least is deformable axially. Various typical valves such as porcine pericardium or the like may meet this requirement. In addition, making the skirt with same material as the valve can omit necessary processes such as verifying the biological nature of the material as the material of the valve have already well developed, facilitating the promotion and implementation of the stent apparatus.

The pulling string of the stent apparatus with skirt for preventing peripheral leakage serves as a driving mechanism for driving the skirt to transform into the stacked configuration, and operates in cooperation with the deformation of the stent during release.

Due to the pulling string is applied as the driving mechanism in the preferred embodiment of the present invention, there are no such strict requirements for the engagement between the skirt and the outer periphery of the stent as the skirt can in any event be pulled and stacked by means of the pulling string.

Optionally, in a released condition of the stent, the skirt loosely fits, conforms or is tightly mounted on the outer periphery of the stent.

The loosely fitting, conforming, or being tightly mounted are defined based on the tightness degree of the engagement between the skirt and the stent, wherein the loosely fitting means that the skirt is engaged relatively loosely with the outer periphery of the stent and there is a certain amount of floating space for the skirt, the conforming means that the skirt is exactly conforming with the outer periphery of the stent with the free floating limited, and without being tightened too much with respect to each other, and being tightly mounted means that the skirt constrains on the outer periphery of the stent under the expanding force of the stent with the elasticity of the skirt or the stent itself.

It is necessary that the stent will expand radially after being released into the human body. The pulling string of the present invention operates in cooperation with the deforming of the stent, during radial deforming of the stent, a force will be generated and transmitted to the pulling string which will in turn drive the skirt to be pulled and folded. A pulling force is transmitted via the flexible pulling string from the stent to the skirt. The diameter of the stent increases when it is released. Two points on the stent that are located close to each other in the circumferential direction before the stent is released will move away from each other after the stent is released. Based on this principle, the pulling string pulls the skirt by transmission of the pulling force.

The pulling string comprises a driving portion and a force exerting portion, wherein the driving portion operates in cooperation with the stent during release. The force exerting portion is connected with the skirt and pulled by the driving portion during release of the stent to obtain an axial displacement relative to the stent so as to pull the skirt to be stacked.

The driving portion and the force exerting portion, as parts of the pulling string, may be segments of the pulling string with certain lengths, or may be certain points. The driving portion pulls the force exerting portion during the deformation of the stent so as to drive the skirt. The pulling force may be oriented parallel to or inclined to the axial direction of the stent, as long as the pulling force has at least a component in the axial direction which drives the force exerting portion to move axially.

During the release of the stent, the radial expansion of the stent is accompanied by changes in the circumferential direction, i.e., perimeter increasing. The pulling string on the stent/skirt operates in cooperation with the radial expansion of the stent. The axial gathering corresponding to the circumferential expansion of the pulling string will drive the skirt to be folded and stacked in the axial direction, and to be further thickened around the outer periphery of the stent, so as to form the annular peripheral leakage occluder.

The length of the pulling string is fixed in both the compressed configuration of the stent before being released and the expanded configuration of the stent after being released. In the expanded configuration of the stent, the pulling string is expanded radially with the expansion of the stent. The axial extension length of the pulling string will be shortened along with the increase of the circumferential extension length of the pulling string, which will simultaneously drive the skirt to be folded axially and transformed into the stacked configuration.

In the fully expanded configuration of the stent, the pulling string maintains the skirt in the folded and stacked configuration.

During preparing of the stent apparatus, the skirt is maintained in the folded and stacked configuration by the pulling string, while the stent is in the fully expanded configuration. When forming the stent: a, arranging a prepared skirt around an outer periphery of the stent in a released configuration; b, threading pulling string through the skirt according to a preset course; c, pulling the pulling string to drive the skirt into a stacked configuration; and d, fixing ends of the pulling string.

In the folded and stacked configuration of the skirt, the pulling string extends circumferentially, preferably, the pulling string is tensioned.

The pulling string extending or being tensioned or being tightened circumferentially does not prevent the stent from expanding.

In the compressed configuration of the stent before being released, the skirt extends axially, and the pulling string extends axially and does not extend beyond the bottom side of the skirt.

The driving portion and the force exerting portion of the pulling string, as parts of the pulling string, may be segments of the pulling string with certain lengths, or may be certain points. As the stent expands and deforms, the circumferential span of the driving portion increases, and the length of the pulling string in the circumferential direction increases. Accordingly, the force exerting portion interacting with the skirt is pulled to axially move closer thereto, thereby driving the skirt to be folded.

In the compressed configuration of the stent, i.e., in the unfolded configuration of the skirt, the pulling string should be straightened as much as possible or slightly tightened between the two bent points (under the guidance of the stent or the skirt), at least neither being loosened nor extending beyond the axial side of the skirt, in order to make a timely response to the deformation of the stent being released so as to render the skirt to be pulled axially. Otherwise, the loosened part of the pulling string will delay or even offset the deformation of the stent, which will affect the effect of pulling the skirt. In the expanded configuration of the stent, the length of the pulling string should not be too short to constrain the stent tightly and prevent the stent from expanding.

The driving portion and the force exerting portion are relative concepts, which are named just for the convenience of illustrating the functions. In other words, it is assumed that only the circumferential span of the driving portions changes, the force exerting portion will be driven and the axial position thereof will be changed. Vice versa, when the circumferential span of force exerting portions changes, the associated driving portion will be driven and the axial position thereof will be changed, in which case, the functions of the driving portion and the force exerting portion are interchanged. In practice, different parts of the pulling string, or the pulling string and the stent operate in cooperative with each other anywhere. Different parts of the pulling string may be considered either as the driving portion or as the force exerting portion from different perspectives, that is, the parts may play dual role. Therefore, although different parts of the pulling string are specifically named, there is no additional limitation to the extension or threading manner of the pulling string, instead, only the cooperation relationship between different parts of the pulling string, and the cooperation relationship between the pulling string and the skirt are emphasized.

Preferably, the driving portion is connected with the stent, and the junction portion is completely or partially located within the overlapping area of the skirt and the stent in the axial direction. Alternatively, the junction portion is completely offset from the overlapping area of the skirt and the stent in the axial direction.

The junction portion of the driving portion and the stent may be exposed outside the skirt or surrounded by the skirt, which may be partially.

Preferably, the driving portion has a circumferential span which changes after the stent is released with respect to that before the stent being released. At least one point of the driving portion acts as the force exerting portion or is connected with the force exerting portion, which obtains an axial displacement relative to the stent with the changing of the circumferential span of the driving portion.

Preferably, at least one section of the skirt in the axial direction is configured as a floating section around the outer periphery of the stent, and the floating section has a stacked configuration resulted from the pull of the pulling string.

In order to make the skirt be deformable in the axial direction and thus be folded and stacked, at least a section of the skirt is required to be axially movable relative to the outer periphery of the stent. The floating section referred to in the present invention may be regarded as an axial floating section, which is axially movable and not necessary to be circumferentially movable. In order to guide the axial movement of the floating section, it is possible to provide a guide mechanism. Therefore, the circumferential movement is not necessarily related to the axial movement. Actually, at least a part of the skirt is circumferentially movable. The floating section generally may be provided with or without circumferentially positioning.

Preferably, one end of the driving portion is connected with a fixed point of the stent, and the driving portion is connected with the force exerting portion after passing through a turning point of the stent. There is a relative circumferential displacement between the fixed point and the turning point after the stent is released with respect to that before the stent being released.

The fixed point and the turning point of the pulling string may be located below the floating section, and therefore the top edge of the floating section is folded towards the bottom edge thereof. Alternatively, the fixed point and the turning point of the pulling string may be located above the floating section, and then the bottom edge of the floating section is folded towards the top edge thereof.

After the stent is released and expanded, the circumferential distance between the fixed point and the turning point will increase so that the other end of the pulling string will pull the top edge of the floating section to move downwards or pull the bottom edge of the floating section to move upwards.

Preferably, there are 2 to 8 pulling strings which are circumferentially arranged around the stent.

Appropriate number of pulling strings may pull the top edge of the floating section moving downwards simultaneously and evenly or pull the bottom edge of the floating section moving upwards evenly and simultaneously.

The pulling string maybe made of surgical suture, elastic thread, nickel-titanium alloy wire, or the like, and preferably be made of the suture connecting the fixing band and the stent.

Preferably, the pulling strings are connected one another circumferentially and in cooperation with each other.

Preferably, the stent is provided with a guiding hole for defining the turning point.

The turning point is provided inside the guiding hole. The pulling string threads through the guiding hole in such a manner that the movement of the pulling string causes axial pulling thereof when one end of the pulling string moves along with the stent.

The guiding hole may be additionally provided on an original stent, or an inherent space formed in the original stent, or provided by a ring additionally provided and attached to the stent.

As further preferred, one or more threading holes are provided on the floating section in the axial direction. The pulling string threads into and out of the floating section through the one or more threading holes after passing through the turning point.

Threading into and out of the floating section through the threading holes on the one hand defines the course and the pulling direction of the pulling string and thus facilitates the stack of the floating section, on the other hand defines the stacking manner and the stack layers.

Preferably, each pulling string threads through 1 to 6 threading holes which are aligned with each other or arranged in a staggered manner in the circumferential direction.

The arrangement manner of threading holes of aligning with each other or in a staggered manner determines the extending direction of the pulling string and the specific stacking configuration as well, resulting in a floating section which may be twisted or shifted circumferentially for example.

Preferably, at least one threading hole is provided on the floating section adjacent to the end nodes of the stent, which prevents the skirt from being snagged on by the end nodes of the stent and failure to be stacked.

Preferably, the stent has a cylindrical meshed structure, and a circumferential distance between the fixed point and the turning point of the stent corresponding to the pulling string spans 1 to 4 cells of the stent.

Preferably, the stent has a cylindrical meshed structure, and the circumferential distance between the fixed point and the turning point of the stent corresponding to the pulling string spans N-1 cells, wherein N denotes the number of the cells of the stent in the circumference where the fixed point locates. The floating section transforms from the unfolded configuration to the stacked configuration with the bottom edge thereof pulled upwards in a certain distance, which depends on the distance variation between the fixed point and the turning point during release of the stent. The axial distance variation of the side wall of the stent is significant, which can be converted to the circumferential span of the cells.

Preferably, the fixed point and the turning point are aligned with each other in a same axial level, or arranged in a staggered manner in the axial direction.

When the fixed point and the turning point are aligned to each other in the same axial level, the pulling string extends circumferentially between the fixed point and the turning point.

When the fixed point and the turning point are arranged in a staggered manner in the axial direction, the pulling string extends inclinedly between the fixed point and the turning point relative to a plane perpendicular to the axial direction, with a component in the circumferential direction.

As further preferred, the fixed point and the turning point are arranged in a staggered manner in the axial direction, which can avoid interference with the floating section and partially radially thickening of the floating section, facilitating the loading and release of the valve replacement device.

In the case that the bottom edge of the floating section is pulled upwards, that is, the end of the floating section released first moves towards the other end released later, in a preferred manner, the end of the pulling string distant from the fixed point is connected at the end of the floating section released first, and the turning point is located at or adjacent to the other end of the floating section released later, and the axial position of the fixed point is further away from the other end of the floating section than the turning point.

In the case that the top edge of the floating section is pulled downwards, that is, the end of the floating section released first moves towards the other end released later, in a preferred manner, the end of the pulling string distant from the fixed point is connected at the other end of the floating section released later, and the turning point is located at or adjacent to the end of the floating section released first, and the axial position of the fixed point is further away from the end of the floating section than the turning point.

Preferably, the pulling string threads circumferentially between the skirt and the stent.

In order to facilitate the skirt to transform into the stacked configuration, a preferred driving mechanism is provided in the invention, in which a pulling string extending circumferentially and threading or being fixed between the stent and the skirt is provided to pull the skirt.

The driving portions of the pulling string may be a plurality of spaced sections or points, which are fixed or movably thread through the stent, and may also thread through the skirt at corresponding positions thereof while threading through the stent.

The force exerting portions of the pulling string may be a plurality of spaced sections or points, which are fixed or movably thread through the skirt.

Preferably, the pulling string undulates in the axial direction of the stent while extending in the circumferential direction of the stent and thus form a wave-like configuration.

The pulling string forms an undulating structure while threading along the circumferential direction. The overall pulling string will be subjected to a radially expanding force of the stent being released, and the shape of the undulating structure will be changed to a certain extent, thereby driving the skirt to be axially folded. The pulling string has an greater undulation after the stent is released than the undulation the pulling string has before the stent being released.

Preferably, the pulling string extends circumferentially along the stent for a perimeter thereof.

Preferably, the driving portions form the peaks and are fixed on the stent, and the force exerting portions form the valleys and movably thread through the skirt

The peaks and valleys are relative concepts in this context, which are intended to illustrate the extreme position or turn-back position of the pulling string in the axial direction. The peaks can be aligned with each other or offset from to each other in the axial level. The same applies to the valleys.

In the preferred manner, the peaks are fixed on the stent, and the valleys movably thread through the skirt. The distance between two adjacent peaks would increase during the release of the stent, while the length of the pulling string which extends between the two adjacent peaks and the valley located between the two adjacent peaks is fixed, thus the valley will be moved towards a line connecting the two adjacent peaks, thereby achieving the axial movement of the skirt until the skirt being generally folded to the stacked configuration.

Preferably, the first portions are the peaks and movably thread through the stent, and the second portions are the valleys and movably thread through the skirt.

In the preferred manner, the peaks may movably thread through the stent via the gaps in the grids of the meshed stent or through holes of the stent or the like, for example. The valleys movably thread through the skirt. The distance between two adjacent peaks will increase during the release of the stent, as the diameter of the stent would increase. However, the overall length of the pulling string is fixed, and therefore the height between the peaks and valleys would be reduced, that is, the undulation between the peaks and valleys becomes more flat. As the axial positions of the peaks are limit by the stent, the valleys are moved close towards the peaks, thereby achieving the axial movement of the skirt until the skirt being generally folded to the stacked configuration.

Preferably, the first portions are the peaks and fixed on or movably thread through the stent, and the second portions are the valleys and fixed to the skirt.

Similarly, the valleys are pulled by the pulling string during the release of the stent, and the skirt is thus also be pulled since the valleys are fixed on the skirt.

Different peaks can be connected to the stent in a same way or different manners. Similarly, different valleys can be connected to the stent in a same way or different manners. For example, one of two adjacent peaks is fixed on the stent, and the other movably threads through the stent. In other words, the pulling string may have an undulating structure with similar wave shapes, but with the peaks and valleys connected in different manners.

Preferably, there are at least two pulling strings, and the peaks and valleys of the two pulling strings are arranged in a staggered manner or are spaced to each other.

A plurality of pulling strings may be provided, each of which surrounds the stent for a perimeter thereof. The plurality of pulling strings are arranged one after another in the axial direction. With the respective undulation structure formed thereof, the pulling strings may be arranged in a staggered manner along the axial direction.

Appropriate staggered manner may facilitate to control the stacking of the skirt, thereby improving the sealing effect.

Preferably, within one wave of the undulation structure, the axial height of the wave is greater than the circumferential length of the wave, which is able to improve the pulling performance, and results in a greater axial displacement thereof under a certain radial deformation of the stent.

Preferably, the skirt is provided with a plurality of cutting areas at least at one axial side thereof which are arranged in the circumferential direction.

Before operation, it is required for the prosthetic heart valve replacement device to be compressed radially and loaded in a delivery system, such as loaded in a sheath, and then delivered into the human body via blood vessels for release. In order to improve the compliance and the capacity through in the human body and reduce the friction on the vessel wall, it is preferred for the whole device to have a smaller diameter after loading and before release. During the loading of the stent being radially compressed, the ends of the skirt which surrounds the outer periphery of the stent will be gathered and disordered, and are difficult to neaten to conform around the outer periphery of the stent. On one hand, it is difficult for the skirt to enter into the sheath. On the other hand, the skirt may be damaged or even fallen off the stent at the end of the loading, as the resistance of the end of the skirt being gathered into the sheath is too large. In order to solve the problem of radial gathering and reduce the size during loading, the skirt of the present invention is partially cut at positions adjacent to the axial ends thereof to reduce the thickness thereof when being gathered, facilitating the loading and delivery.

Preferably, the cutting areas are hollow structures.

The hollow structures, as one kind of the cutting areas, may maintain the integrity of the skirt, and contributes to standardized cutting shapes by processes such as punching or laser cutting or the like, facilitating to control error and mass production.

Preferably, within each cutting area, the hollow structure comprises a plurality of through holes arranged along the axial direction of the stent.

The hollow structure comprising through holes arranged sequentially leaves connection portions between the through holes, which is beneficial to the overall shaping of the peripheral leakage occluder and maintains necessary pulling effect between different areas.

The shape of the through holes may be various, such as circular, elliptical, polygonal, or crescent-shaped or the like. The shapes of the through holes in the same row may be the same or different.

Preferably, different cutting areas have the same hollow structure.

The design of the same hollow structure contributes to mass production. In addition, the force on the skirt in the circumferential direction may distribute more evenly, and the resulted annularly stacked configuration has a more uniform profile.

As another preferred manner, a stacked configuration with an approximate uniform profile may also be obtained if the size of each hollow area is small enough.

The plurality of cutting areas arranged in the circumferential direction mainly means that the cutting areas are distributed in the whole circumference. Although the cutting areas may be spaced with a certain distance, they are not concentrated in a particular local area in the circumferential direction.

A plurality of cutting areas may have or not have clear boundaries. For example, in the case of a plurality of through holes densely arranged, the through holes may be distributed randomly or dispersedly, so it is not necessary for the cutting areas to have clear boundaries among there. Even in the case of clearly regular structural units, the boundary between the units may be the generatrix of the skirt, or may be distributed in a certain angle relative to the generatrix, such as be distributed spirally.

Preferably, among the through holes of the same cutting area, the closer to the corresponding side edge of the skirt, the larger size the through hole has.

The cutting area is located at the top or bottom of the skirt. Taking the cutting area at the bottom for an example, the closer to the bottom edge of the skirt, the larger size the through hole has. In other words, the closer to the edge of the skirt, the smaller area the remaining portion of the skirt has, which facilitates to the attachment of the skirt to the outer periphery of the stent and the loading of the stent apparatus.

Preferably, the inner edge of the through hole is smoothly curved.

The strength of the skirt is limited. If there are clear corners on the inner edge after forming the through hole, there will be a localized stress concentration during the deformation of the skirt, resulting a risk of being torn. The smoothly curved inner edge may greatly reduce such risk and improve the safety in use.

Preferably, at least one axial side of the skirt is configured as a tooth-shaped structure having a plurality of teeth arranged in the circumferential direction, with one of the cutting areas defined between adjacent teeth.

As another preferred cutting manners, in the present invention, the skirt is cut at least at one axial side thereof, and the remaining portions at the edge are tooth-shaped with teeth distributed circumferentially. In this case, the skirt being radially compressed will have a smaller outer diameter at the cut end thereof, which facilitates the loading of the stent apparatus.

Moreover, by using the tooth-shaped cutting structure, the edge of the skirt is opened, which can avoid intertwining or interference of the skirt with the stent or the pulling string, facilitating the release of the skirt.

Optionally, the teeth of the tooth-shaped structure have the same or different shapes.

Optionally, the shape of each tooth is one or any combination of a triangle, a rectangle, a trapezoid, and a semicircle.

Within the tooth-shaped structure, the shapes of the teeth are not strictly limited. The tooth-shaped structure may also be combined with the hollow structure such that the remaining area at the end of the skirt may be reduced. The configuration of the tooth includes both the geometrical shape and the size. For example, all the teeth may be triangular teeth, while they are distinguished as large teeth and small teeth and arranged alternately. Alternatively, rectangular teeth and triangular teeth may be provided and arranged alternately.

Preferably, the teeth have the same configuration are evenly arranged in the circumferential direction.

The teeth with the same configuration mean that they are evenly arranged in the circumferential direction. Although both of the axial ends of the skirt may have tooth-shaped structures, the tooth-shaped structures at both of the axial ends may be the same or different. The even distribution of the teeth at the same end of the skirt renders the skirt subject to force distributed evenly in the circumferential direction, and be well adaptable in the circumferential direction, without special positioning means.

As further preferred, both sides of the triangle tooth are inclined relative to the axial direction of the stent. Alternatively, one side is parallel to the axial direction of the stent, and the other side is inclined relative to the axial direction of the stent.

Preferably, before the stent is released, the side of the skirt to be released first extends beyond the stent in the axial direction, and it is the tooth-shaped structure that extends beyond the stent.

In order to obtain a peripheral leakage occluder with sufficient volume, the skirt should have sufficient axial length. However, if the skirt is so long, especially before the stent is released, that the side of the skirt to be released first extends beyond the stent in the axial direction, the stent will be released after the skirt and may roll outwards and snag or even pierce the inner wall of the skirt, preventing the skirt from being folded axially. This situation can be avoided if the side of the skirt adopts the tooth-shaped structure.

The portion of the skirt that extends beyond the stent is released before the stent. The stent during release is able to smoothly turn out from the cutting areas. Even if some teeth of the skirt may cover the stent, they will adaptively avoid the stent as the teeth have a high degree of freedom. As further preferred, the tooth root of the tooth-shaped structure does not extend beyond the bottom end of the stent.

If the tooth root slightly extends beyond the edge of the corresponding side of the stent, the above risks still exist. Taking the bottom side of the stent to be released first as an example, the top edge of the skirt is fixed on the stent. In the unfolded configuration, the bottom side of the skirt extends axially beyond the bottom edge of the stent, and the portion of the skirt that extends beyond the bottom of the stent is the tooth-shaped structure.

Preferably, the side of the skirt with the cutting areas floats around the outer periphery of the stent, and a limiting string may be provided between the floating side of the skirt and the stent for limiting the maximum axial movement of the floating side of the skirt.

Preferably, the floating side of the skirt is configured as a tooth-shaped structure extending having a plurality of teeth arranged in the circumferential direction, with one of the cutting areas defined between adjacent teeth. The limiting string is connected at the top tip of each tooth.

The movement of the teeth may be restrained and limited by means of the pulling line, especially in the case of a long-tooth-shaped structure. For example, the bottom of the skirt may have the tooth-shaped structure and floats around the outer periphery of the stent, and the limiting string is provided between the top tips of the teeth and the stent for limiting the maximum axial movement of the floating side of the skirt.

Similar to the tooth-shaped structure, the hollow structure may also be provided with a limiting string. In addition, even if the skirt does not have cutting areas, the limiting string may also be applied to control the axial movement of the skirt as required. The limiting string may also be connected at the axial middle portion of the skirt, and affect the shaping and configuration of the peripheral leakage occluder to a certain extent.

Preferably, the side of the skirt with the cutting areas is fixed on the outer periphery of the stent.

The side of the skirt with the cutting areas may also be fixed on the stent, which usually serves as the side of the side of the skirt to be released later. The loading process is just reversed, with the cutting areas fixed entering into the sheath first. The cutting areas may be in the form of hollow structure or cutting structure at the edge.

Preferably, one axial side of the skirt is configured as a tooth-shaped structure having a plurality of teeth arranged in the circumferential direction, with one of the cutting areas defined between adjacent teeth.

Within the tooth-shaped structure, a vertex of each tooth is fixed on the stent, or the vertex and edges of each tooth are fixed on the stent.

The tooth-shaped structure is formed by cutting materials at the edge, which may be fixed only at the vertexes of the teeth. In this situation, the fixing band is stitched discontinuously with a plurality of stitching portions with intervals.

If the edges of the teeth are also stitched on the stent, the fixing band is fixed in a continuously stitching manner. Compared to the manner of stitching discontinuously with intervals, the manner of stitching continuously may bring a stronger connection strength, but result in additional radial expansion to a certain extent.

Preferably, the stent has a meshed structure having a plurality of grids, and the shape of teeth is corresponding to that of the grids at a corresponding portion of the stent.

A tooth having the same shape as the grid further facilitates to the stitching, especially to the stitching continuously and densely, which, on one hand, avoids unnecessary parts of the skirt, and on the other hand, ensures the sufficient attachment for the edges of the teeth on the meshed stent.

Preferably, the skirt is annularly provided with a pushing pocket at an inflow side of normal blood flow for facilitating the skirt to transform into the stacked configuration, and the pushing pocket has at least one inlet for allowing blood to flow in, by means of which the pushing pocket is self-expandable to push the skirt.

The pushing pocket is made of flexible material, which may be made of the same material as the skirt or even be a part of the skirt. The pushing pocket is fixed to the outer periphery of the stent in a stitching manner, and has a double-layer structure, via which it may be filled with blood and thus be expanded radially.

The inlet for allowing normal blood flow to flow in faces away from the annulus, i.e.,, facing towards the normal blood flow, and functions to receive the normal blood flow. After the blood flows into the pushing pocket, the pushing pocket self-expands to assist the skirt to move axially and thus be folded.

Preferably, the bottom edge of the skirt in the unfolded configuration extends beyond the pushing pocket.

The pushing pocket after being expanded contacts and pushes the skirt. If the pushing pocket and the skirt are not in contact, the pushing effect may be unsatisfied. More preferably, an extension portion of the bottom edge of the skirt in the unfolded configuration covers the pushing pocket fully or partially. In this way, the radial expansion of the pushing pocket may be well transmitted to the skirt.

Preferably, one side of the pushing pocket is closed, and the other side is provided with a plurality of stitching portions fixed to the stent. The stitching portions are spaced from each other with an inlet for allowing normal blood to flow in defined between two adjacent stitching portions.

Preferably, a plurality of inlets for allowing blood to flow in are provided which are evenly arranged in the circumferential direction.

The plurality of inlets for allowing blood to flow in arranged in the circumferential direction are formed by stitching with intervals, that is, the areas that are not closed by stitching serve as the inlets for allowing blood to flow in. The plurality of inlets for allowing blood to flow in enable the pushing pocket to expand uniformly with a significant receiving performance.

The pushing pocket is: formed separately; or formed integrally with the coverage membrane inside of the stent as a single piece. Alternatively, a portion of the pushing pocket is formed by the material of the coverage membrane inside of the stent.

The pushing pocket may be formed in the following manners.

1. The pushing pocket is separately provided relative to the material of the skirt . Since the pushing pocket is provided as a double-layer structure, the inner and outer layers may be integrally formed as a single piece, which are connected by a bend at the top side. Alternatively, the inner and outer layers are separately formed, which are connected and sealed by stitching the tops there between. At the bottom of the pushing pocket, the top edge of the inner layer of the pushing pocket is generally continuously stitched on the outer periphery of the stent by stitching densely, and the outer layer is stitched on the stent discontinuously with intervals to form the plurality of inlets for allowing blood to flow in.

2. The pushing pocket and the coverage membrane inside of the stent are integrally formed as a single piece, in which the pushing pocket is formed by turning outwards and axially folding the coverage membrane inside of the stent, and the inner and outer layers may be stitched together by the means as described above.

3. A portion of the pushing pocket is formed by the material of the coverage membrane inside of the stent. The pushing pocket is provided as a double-layer structure including an inner layer and an outer layer. The inner layer and the outer layer are closed at the top, and the inlets for allowing blood to flow in are formed at the bottom. Either one of the inner and outer layers may be formed by the material of the coverage membrane inside or outside of the stent.

For example, the inner layer of the pushing pocket is integrally formed with the skirt as a single piece, and only the outer layer is additionally provided and stitched on the periphery of the skirt.

Alternatively, the outer layer of the pushing pocket is integrally formed with the skirt as a single piece. An inner layer is first provided and stitched, and then the top of the skirt is attached to the outer periphery of the inner layer and thus forms the outer layer of the pushing pocket. The plurality of inlets for allowing blood to flow in are formed by cutting at appropriate positions.

The present invention also provides a heart valve comprising a stent and a valve provided in the stent, wherein the stent is the stent apparatus with skirt for preventing peripheral leakage according to the invention.

The valve in the stent apparatus can form an one-way blood channel, the valve in the stent and the skirt located around the outer periphery of the stent can be made of the same material or different materials.

The present disclosure also provides a processing method for the stent apparatus with skirt for preventing peripheral leakage, including the steps of:

  a. arranging a prepared skirt around an outer periphery of the stent in a released configuration;
  b. threading a pulling string through the skirt according to a preset course;
  c. pulling the pulling string to drive the skirt to transform into the stacked configuration; and
  d. fixing ends of the pulling string.

The length of the pulling string is related to the size of the skirt after being expanded relative to the stent, the perimeter of the stent after expanding, and the size and number of peripheral leakage occluder. Before the stent is loaded and compressed, the skirt has been stitched on the stent. Since the stent is generally in an expanded configuration during stitching, the configuration of the skirt after being stitched and before being loaded is basically the same as that of the skirt after the stent is expanded in the human body, i.e., the stacked configuration of the skirt after the stent is expanded. Therefore, it may be regarded that the process of stitching the skirt on the stent is a process to predefine the configuration of the skirt. In order to reduce the radial dimension during loading, the predefined skirt is axially unfolded. After the stent is expanded in the human body, the skirt is able to be restored to the stacked configuration as predefined.

In order to facilitate the threading of the pulling string, preferably, in the step of a, the skirt is flattened around the outer periphery of the stent in the expanded configuration.

The step a further comprises fixing at least a part of the skirt on the stent.

The step a further comprises stitching the skirt as a cylindrical structure which is closed in the circumferential direction before or after the skirt is surrounded around the outer periphery of the stent.

With respect to the manner of fixing the end, preferably, in the step d, the pulling string comprise two ends which are connected with each other. Other than being connected with each other, the ends may also be connected by themselves. The nodes of the ends is just to prevent the pulling string from falling off from the skirt, which is not necessary or strictly limited for driving the skirt to transform into the stacked configuration. Therefore, it should be considered as an equivalent even tying the ends on the stent.

The disclosure also provides a folding method for a skirt of a stent apparatus at an implantation site of the stent, wherein the stent apparatus is the stent apparatus with skirt for preventing peripheral leakage of the invention, and the skirt comprising:

an unfolded configuration loaded in a sheath before the stent is released, in which the skirt is axially unfolded and surrounding an outer periphery of the stent before being released; and

a stacked configuration during or after the release of the stent, in which the skirt is folded and stacked in an axial direction of the stent after being released and forms an annular peripheral leakage occluder;

the stent apparatus is further provided with a pulling string that only threads through the skirt, and the pulling string operates in cooperation with radial deforming of the stent during release so as to drive the skirt to transform into the stacked configuration.

The specific structures of the stent apparatus, the skirt and pulling string involved in the folding method for a skirt may be referred to the detail disclosures according to the present invention, and will not be repeated here.

The peripheral leakage prevention means provided by the present invention provides an implanting stent more suitable with the inner wall of the blood vessels, so that the stent would not easily be migrated and is more stable, with expanded applicable population, lower additional surgical risks, and reduced peripheral leakage, thrombi and other complications. Better hemodynamic performance is provided, the coverage function of endothelial cells of the host is enhanced, the probability of occurrence of endocarditis is lowered, and the normal blood supply function of the heart and blood vessels is recovered

**BRIEF DESCRIPTION OF THE DRAWINGS**

Fig. 1a is a schematic structural view of a traditional aortic stent.

Fig. 2a is a schematic structural view of a stent apparatus according to a first embodiment of the present disclosure, showing a stent thereof before being released.

Fig. 2b is a schematic structural view of the stent apparatus according to the first embodiment, showing a pulling unit before being released.

Fig. 2c is a schematic structural view of the stent apparatus according to the first embodiment, showing the pulling unit before being released

Fig. 2d is a schematic structural view of the stent apparatus according to the first embodiment, showing the stent after being released.

Fig. 2e is a schematic structural view of a stent apparatus, the pulling unit of which has a different circumferential span compared to the first embodiment.

Fig. 2f is another schematic structural view of a stent apparatus, the pulling unit of which has a different circumferential span compared to the first embodiment.

Fig. 2g is a further schematic structural view of a stent apparatus, the pulling unit of which has a different circumferential span compared to the first embodiment.

Fig. 2h is a schematic structural view of a stent compared to the first embodiment.

Fig. 3 is a schematic structural view of a stent apparatus according to a second embodiment of the present disclosure.

Fig. 4 is a schematic structural view of a pulling unit of the stent apparatus according to the second embodiment.

Fig. 5a is a schematic structural view of a pulling unit of a stent apparatus according to a third embodiment of the present disclosure, showing the pulling unit before being released;

Fig. tb is a schematic structural view of a pulling unit of a stent apparatus according to the third embodiment, showing the pulling unit after being released;

Fig. 6 is a schematic structural view of a pulling unit of a stent apparatus according to a fourth embodiment of the present disclosure.

Fig. 7a is a schematic structural view of a stent apparatus according to a fifth embodiment of the present disclosure, showing a stent thereof before being released.

Fig. 7b illustrates how a pulling string of the stent apparatus according to the fifth embodiment threads through a skirt.

Fig. 7c is a schematic structural view of the pulling unit of the stent apparatus according to the fifth embodiment, showing the pulling unit before being released.

Fig. 7d is a schematic structural view of the pulling unit of the stent apparatus according to the fifth embodiment, showing the pulling unit after being released.

Fig. 7e is a schematic structural view of the stent apparatus according to the fifth embodiment, showing the stent after being released.

Fig. 8 is a schematic structural view a stent apparatus according to a sixth embodiment of the present disclosure.

Fig. 9 is a schematic structural view of a pulling unit of a stent apparatus according to a seventh embodiment of the present disclosure showing the pulling unit before being released;

Fig. 10a is a schematic structural view of a pulling unit of a stent apparatus according to a eighth embodiment of the present disclosure, showing the pulling unit before being released;

Fig. 10b shows two skirts adopting different driving mechanisms;

Fig. 10c shows two skirts adopting further different driving mechanisms;

Fig. 11 is a schematic structural view of a pulling unit of a stent apparatus according to a ninth embodiment of the present disclosure, showing the pulling unit before being released;

Fig. 12 is a schematic structural view of a pulling unit in a stent apparatus according to a tenth embodiment of the present disclosure showing the pulling unit before being released;

Fig. 13 is a schematic structural view of a pulling unit of a stent apparatus according to a eleventh embodiment of the present disclosure, showing the pulling unit before being released;

Fig. 14a is a schematic structural view of a stent apparatus according to a twelfth embodiment of the present disclosure, showing a stent thereof before being released.

Fig. 14b illustrates how a pulling string of the stent apparatus according to the twelfth embodiment threads through a skirt.

Fig. 14c is a schematic structural view of the stent apparatus according to the twelfth embodiment, showing the stent after being released.

Fig. 15 illustrates how a pulling string of a stent apparatus according to a thirteenth embodiment threads through a skirt.

Fig. 16a illustrates how a pulling string of a stent apparatus according to a fourteenth embodiment threads through a skirt.

Fig. 16b illustrates how the pulling string of the stent apparatus according to the fourteenth embodiment threads into and out of the skirt.

Fig. 16c is a schematic structural view showing the skirt of the stent apparatus according to the fourteenth embodiment in an unfolded configuration.

Fig. 16d is a schematic structural view showing the skirt of the stent apparatus according to the fourteenth embodiment in a stacked configuration .

Fig. 17 illustrates how a pulling string of a stent apparatus according to a fifteenth embodiment threads through a skirt.

Fig. 18 illustrates how a pulling string of a stent apparatus according to a sixteenth embodiment threads through a skirt.

Fig. 19a illustrates how the pulling string of the stent apparatus according to the seventeenth embodiment of the present disclosure threads through the skirt.

Fig. 19b shows how the pulling string threads through the skirt, wherein the pulling string has a different perimeter compared to Fig. 19a.

Fig. 19c shows how the pulling string threads through the skirt, wherein the pulling string has a different perimeter compared to Fig. 19a.

Fig. 20 is a schematic structural view of a stent apparatus according to a eighteenth embodiment of the present disclosure.

Fig. 21 is a schematic structural view of a stent apparatus according to a nineteenth embodiment of the present disclosure.

Fig. 22 is a schematic structural view of a stent apparatus according to a twentieth embodiment of the present disclosure.

Fig. 23 is a schematic structural view showing cutting areas of a skirt of a stent apparatus according to a twenty-first embodiment of the present disclosure.

Fig. 24 is a schematic structural view showing cutting areas of a skirt of a stent apparatus according to a twenty-second embodiment of the present disclosure.

Fig. 25 is a schematic structural view showing cutting areas of a skirt of a stent apparatus according to a twenty-third embodiment of the present disclosure.

Fig. 26 is a schematic structural view showing cutting areas of a skirt of a stent apparatus according to a twenty-fourth embodiment of the present disclosure.

Fig. 27 is a schematic structural view showing cutting areas of a skirt of a stent apparatus according to a twenty-fifth embodiment of the present disclosure.

Fig. 28a is a schematic structural view of a stent apparatus according to a twenty-sixth embodiment of the present disclosure.

Fig. 28b is a schematic structural view showing a part of a blocking pocket of the stent apparatus according to the twenty-sixth embodiment.

Fig. 29 is a schematic structural view of a stent apparatus according to a twenty-seventh embodiment of the present disclosure.

Fig. 30 is a schematic structural view showing a part of a blocking pocket of a stent apparatus according to a twenty-eighth embodiment of the present disclosure.

Fig. 31 is a schematic structural view showing a part of a blocking pocket of a stent apparatus according to a twenty-ninth embodiment of the present disclosure.

Fig. 32a is a schematic diagram showing the relationship between the circumferential distance variation between a first acting portion and a second acting portion and the axial displacement of a force exerting portion according to a thirty embodiment of the present disclosure.

Fig. 32b is a schematic diagram showing the relationship between the circumferential distance variation between the first acting portion and the second acting portion and the axial displacement of the force exerting portion according to the thirty embodiment after the shape of a pulling unit is changed.

Fig. 32c is a schematic diagram showing the thickness of a peripheral leakage occluder after a skirt is folded according to the thirty embodiment.

Figs. 33a to 33c are schematic diagrams according to a thirty-first embodiment of the present disclosure.

Fig. 34a is a schematic diagram of a delivery system according to a thirty-second embodiment of the present disclosure.

Fig. 34b is a schematic view according to the thirty-second embodiment, in which a stent with a skirt is in a loaded configuration.

Fig. 34c is a schematic view according to the thirty-second, in which the stent with the skirt is in an intermediate state during release.

Fig. 34d is a schematic view according to the thirty-second, in which the stent with the skirt is in a released configuration.

Fig. 34e is a schematic view according to the thirty-second embodiment, showing the delivery system entering into the aortic valve.

Fig. 34f is a schematic view according to the thirty-second embodiment, in which the stent in the delivery system is being released to an intermediate state at the aortic valve.

Fig. 34g is a schematic view according to the thirty-second embodiment, in which the stent in the delivery system is fully released at the aortic valve.

## DESCRIPTION OF THE EMBODIMENTS

In order to make the technical solutions of the embodiments more apparent and complete, the present invention will be further described in detail below with reference to accompanying drawings and embodiments. It should be understood that the embodiments described are illustrated as a part of the embodiments according to the present invention, but not exhaustive. Any modifications obtained by those skilled in the art based on the descriptions of the embodiments of the present invention without inventive efforts are also within the scope of the present invention.

In order to clearly describe and illustrate the embodiments of the present application, one or more figures may be referred to, while the additional details or illustrations for describing the figures should not be regarded as any limitations to any one of the invention, the embodiments described hereinafter, and the preferred embodiments of the present application.

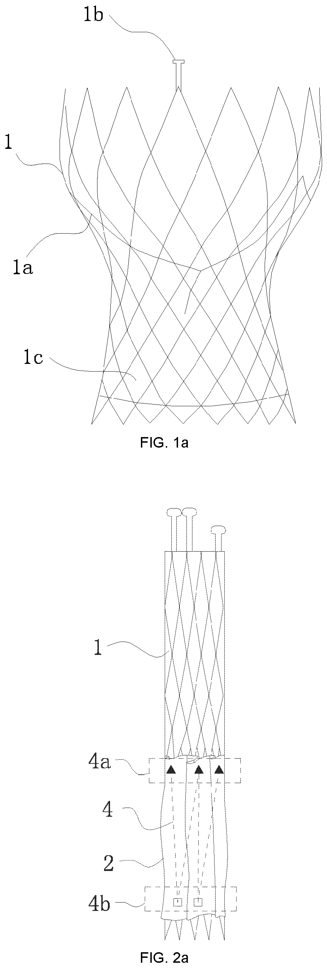

In the stent apparatus with skirt for preventing peripheral leakage according to the present invention, the stent may be any known stent in the art. A distal axial end of the stent after entering into the body is the to be released first, and a proximal end is to be released later. During release of the stent, any axial end thereof may serve as the distal end, with the other end thereof as the proximal end, which depends on physiological structures of different lesion sites in the human body and operating methods of implantation surgeries. In Fig. 1a, an aortic stent including a stent 1 is taken as an example. The stent 1 is made of material such as nickel-titanium alloy. A fixing ear 1b for connecting a delivery system is provided at the top of the stent (referring to the orientations shown in the figures). Depending on applications and requirements of the stent, a valve 1a or an inner coverage membrane 1c may be provided in the stent. The peripheral leakage referred in the present invention is not strictly limited to the aortic valve, but also may occur in other positions in the human body with similar physiological structures.

Unless otherwise specified, in the figures of the following embodiments: solid triangles illustrate that the portions of a pulling string there are fixed to the stent. If the corresponding area of the stent is surrounded by the skirt, the pulling string may thread or not thread through the corresponding portions of the skirt as required; hollow triangles illustrate that the portions of the pulling string there movably thread through the stent. If the corresponding area of the stent is surrounded by the skirt, the pulling string may thread or not thread through the corresponding portions of the skirt as required; solid rectangular boxes illustrate that the portions of a pulling string there are fixed to the skirt and extend around the outer periphery of the stent without threading into the interior of the stent. hollow rectangular boxes illustrate that the portions of the pulling string there movably thread through the skirt and extend around the outer periphery of the stent without threading into the interior of the stent.

First embodiment

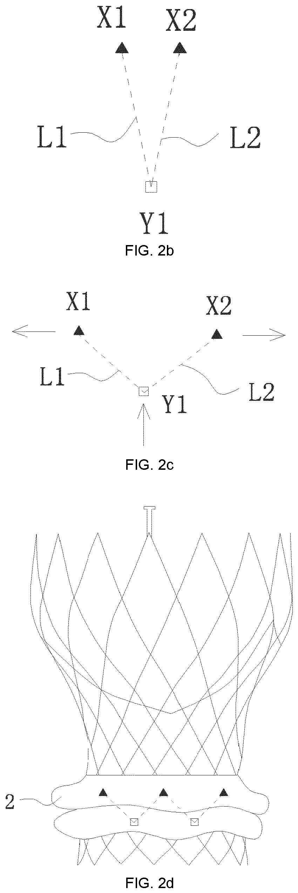

Referring to Figs. 2a to 2d, in the first embodiment, the outer periphery of the stent 1 is provided with a flexible skirt 2. Before the stent 1 is released, the skirt 2 is in an unfolded configuration, which is axially unfolded and surrounding the outer periphery of the stent 1 before release.

In other preferred developments, both ends of the skirt in an unfolded configuration do not extend beyond the corresponding sides of the stent in the axial direction of the stent.

Valves may be provided in the stent, which, depending on the application sites, may be heart valves for example, to prevent perivalvular leakage.

After the stent 1 is released, the skirt 2 is folded and stacked along the axial direction of the released stent 1 into a stacked configuration and forms an annular peripheral leakage occluder. Also provided in this embodiment is a pulling string 4 for driving the skirt 2 to transform into the stacked configuration, which is cooperative with the radial deforming of the stent 1 during release. The pulling string 4 includes a driving portion 4a and a force exerting portion 4b. The driving portion 4a is fixed on the stent in a discontinuous manner. The driving portion 4a is located within the area where the skirt 2 is located and thus stitched and fixed on the stent 1 together with the corresponding part of the skirt 2. On the other hand, the driving portion 4a can be regarded as a fixing band for fixing the skirt 2 relative to the stent 1.

The force exerting portion 4b is connected to the skirt 2 and movably threads through the corresponding portion of the skirt. During the release of the stent 1, the force exerting portion 4b is pulled by the driving portion 4a and has an axial displacement relative to the stent 1 to drag the skirt 2 to be stacked.

In this embodiment, other than the top of the skirt which is connected to the stent through the driving portion 4a, the lower region of the skirt has a large movement degree relative to the stent and thus can be regarded as a floating section around the outer periphery of the stent 1, wherein it is the floating section that forms the stacked configuration after being pulled by the pulling string 4.

The pulling string 4 which serves to act on the floating section includes a plurality of pulling units arranged in the circumferential direction. A portion of the pulling string, or a single pulling string may serve as a single unit to drive the floating section to be folded axially, wherein one example based on the triangular deformation is shown as below.

Shown as Fig. 2b, before release, the driving portion includes a first action portion X1 and a second action portion X2 respectively connected to the skirt, and the force exerting portion Y1 is connected to the skirt.

Referring to Fig. 2c, since the overall length of the portion of the pulling string corresponding to the pulling unit is fixed, that is, the overall length of the sides L1 and L2 of the triangle is fixed. When the stent is being released, the first acting portion X1 and the second acting portion X2 move away from each other in the directions indicated by the respective arrows, and thus the distance between the first acting portion X1 and the second acting portion X2 increases, and the shape of the triangle will change. Therefore, the force exerting portion Y1 as a vertex will move towards a line connecting the first action portion X1 and the second action portion X2 in the direction as indicated by the upward arrow, that is, the force exerting portion Y1 will move axially relative to the stent, and pull the skirt.

In order to obtain a larger deformation for the triangle of each pulling unit, the first acting portion X1 and the second acting portion X2 span one or more cells in the circumferential direction. For example, in this embodiment, the first acting portion X1 and the second acting portion X2 substantially span two cells. The central angle corresponding to the first acting portion X1 and the second acting portion X2 in the circumferential direction after the stent is released is 60 degrees. The pulling units circumferentially cover the entire skirt, however, only two pulling units are shown in the figures, with the others omitted for convenience of illustration

All the pulling units are connected in a wave-like manner with a plurality of waves and each wave having a triangle shape. Along the extension direction of the pulling string, the force exerting portion Y1 is located between the first action portion X1 and the second action portion X2, and the first action portion X1 and the second action portion X2 are opposite to each other (namely, they are located at the same level along the axial direction). Alternatively, pulling units may be arranged in such a manner that they are spaced from each other. In other words, two adjacent pulling units do not share any portion of the pulling thread. For ease of identification, the pulling string is indicated as dashed lines. Actually, the pulling string threads through in such a manner that some sections thereof are arranged outside of the skirt, and some sections thread into the inner side of the skirt via threading holes, and the sections located outside and the sections located inside are arranged alternatively in the circumferential direction.

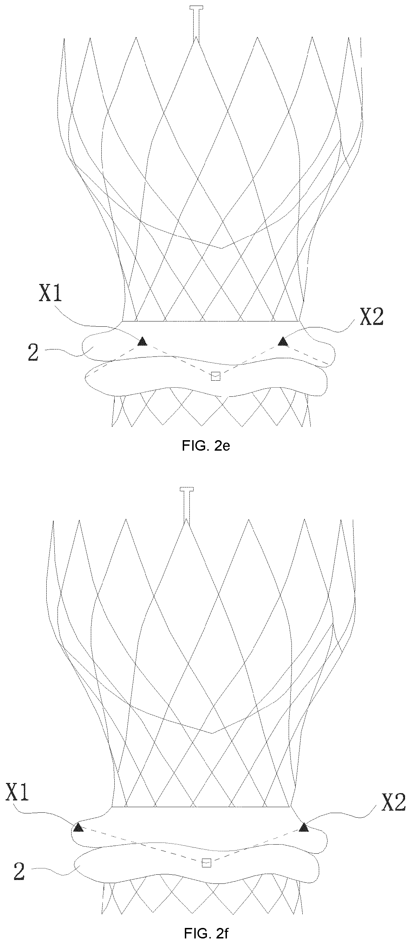

Referring to Fig. 2e, in a further development, within the pulling string on the skirt 2, the central angle corresponding to the first acting portion X1 and the second acting portion X2 in the circumferential direction is 90 degrees.

Referring to Fig. 2f, in a further development, within the pulling string on the skirt 2, the central angle corresponding to the first acting portion X1 and the second acting portion X2 in the circumferential direction is 180 degrees.

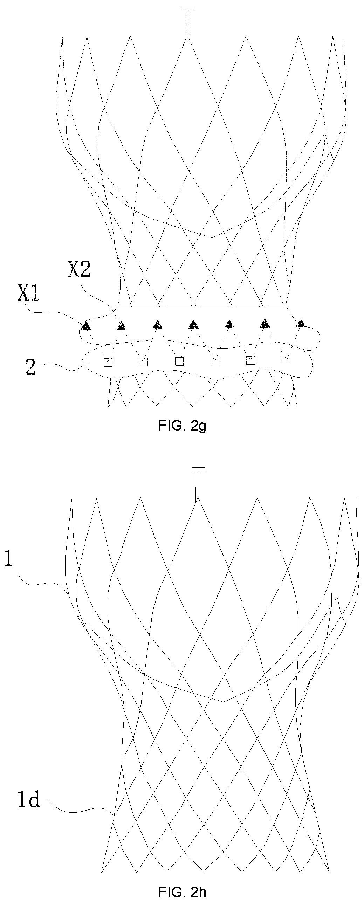

Referring to Fig. 2g, in a further development, within the pulling string on the skirt 2, the central angle corresponding to the first acting portion X1 and the second acting portion X2 in the circumferential direction is 30 degrees.

Referring to Fig. 2h, in another implementation, the bottom of the stent 1 is provided with a flared driving structure 1d, which extends from the middle portion of the stent to the end of the stent (the bottom end). The closer to the bottom end of the stent, the larger diameter the flared driving structure 1d has. In this embodiment, the generatrix of the flared driving structure 1d is substantially straight. The wider side (the bottom end) of the flared driving structure 1d is released first, and the narrow side is released later. Due to such configuration, the flared structure of the stent may operate in cooperation with the skirt during the release of the stent and guides the skirt to stack axially.

The skirt 2 is made of flexible material, such as a porcine pericardium, or a bovine pericardium, or other flexible biocompatible materials, which is configured for constraining the outer periphery of the stent after the stent is released. The skirt is substantially in a cylindrical shape, having a diameter smaller than the outer diameter of the portion of the stent in the released state where the skirt surrounds, such that a constraint force is formed by the skirt onto the stent. During the release of the stent, the stent expands radially and outwards gradually and takes an intermediate configuration with the flared configuration formed at the distal end that is released first, which may guide the flexible skirt to stack in the axial direction.

The processing method of the skirt with the pulling string includes the steps of: arranging a prepared skirt around an outer periphery of the stent in an expanded configuration; threading the pulling string through the skirt according to a preset course; pulling the pulling string to drive the skirt to transform into the stacked configuration; and fixing ends of the pulling string.

The length of the pulling string is related to the size of the skirt after being expanded relative to the stent, the perimeter of the stent after expanding, and the size and number of peripheral leakage occluder. Before the stent is loaded and compressed, the skirt has been stitched on the stent. Since the stent is generally in an expanded configuration during stitching, the configuration of the skirt after being stitched and before being loaded is basically the same as that of the skirt after the stent is expanded in the human body, i.e., the stacked configuration of the skirt after the stent is expanded. Therefore, it may be regarded that the process of stitching the skirt on the stent is a process to predefine the configuration of the skirt. In order to reduce the radial dimension during loading, the predefined skirt is axially unfolded. After the stent is expanded in the human body, the skirt is able to be restored to the stacked configuration as predefined.

In order to facilitate the threading of the pulling string, in the step of a, the skirt is flattened around the outer periphery of the stent in the expanded configuration. It is also possible that at least a part of the skirt may be selectively fixed to the stent according to the connection relationship between the pulling string and stent.

The skirt is stitched as a cylindrical structure which is closed in the circumferential direction before or after the skirt is surrounded around the outer periphery of the stent.

With respect to the manner of fixing the end, in the step d, the pulling string comprise two ends which are connected with each other. Other than being connected with each other, the ends may also be connected by themselves. The nodes of the ends is just to prevent the pulling string from falling off from the skirt, which is not necessary or strictly limited for driving the skirt to transform into the stacked configuration. Therefore, it should be considered as an equivalent even tying the ends on the stent.

Second embodiment

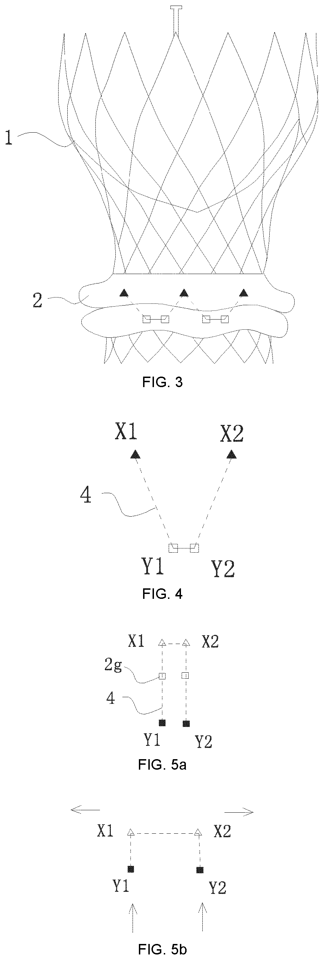

Referring to Figs. 3 and 4, in the second embodiment, the outer periphery of the stent 1 is provided with a flexible skirt 2. Before the stent 1 is released, the skirt 2 is in an unfolded configuration, which is axially unfolded and surrounding the outer periphery of the stent 1 before release. After the stent 1 is released, the skirt 2 is folded and stacked along the axial direction of the released stent 1 into an annular peripheral leakage occluder. Also provided is a pulling string 4 for driving the skirt 2 to transform into the stacked configuration, which operates in cooperation with the radial deformation of the stent 1 during release.

The force exerting portions Y1 and Y2 of the pulling string 4 movably thread through the skirt 2, respectively. The pulling unit is generally in a shape of trapezoid and the deformation of which follows the principle similar to the first embodiment.

During the release of the stent, the perimeter of the stent increases. The first acting portion X1 and the second acting portion X2 move away from each other, and the shape of pulling unit changes. The force exerting portions Y1 and Y2 move towards a line connecting the first acting portion X1 and the second acting portion X2, that is, move axially relative to the stent, so as to pull the skirt.

Third embodiment

Referring to Fig. 5a and Fig. 5b, in this embodiment, the outer periphery of the stent is provided with a flexible skirt. Before the stent is released, the skirt is in an unfolded configuration, which is axially unfolded and surrounding the outer periphery of the stent before release. After the stent is released, the skirt is folded and stacked along the axial direction of the stent after release into a stacked configuration and forms an annular peripheral leakage occluder. Also provided is a pulling string 4 that is configured to drive the skirt into a stacked configuration. The pulling string 4 is cooperative with radial deforming of the stent during release.

In this embodiment, the pulling string 4 movably threads through the skirt at the first acting portion X1 and the second acting portion X2. The two ends of the pulling string 4 are respectively fixed on the skirt 2 as force exerting portions Y1 and Y2.

The entire pulling unit is in a shape of rectangle or trapezoid. During the release of the stent, the perimeter of the stent increases. The first acting portion X1 and the second acting portion X2 move away from each other, and the shape of pulling unit changes. The force exerting portions Y1 and Y2 move towards a line connecting the first acting portion X1 and the second acting portion X2, that is, move axially relative to the stent, so as to pull the skirt.

In the case that the pulling string 4 has a long course, in order to limit the position thereof relative to the skirt or limit the threading direction thereof, a plurality of threading holes 2g may be provided on the skirt based on a preset threading course. such that the pulling string may thread through the plurality of threading holes 2g so as to ensure the pulling performance.

Fourth embodiment

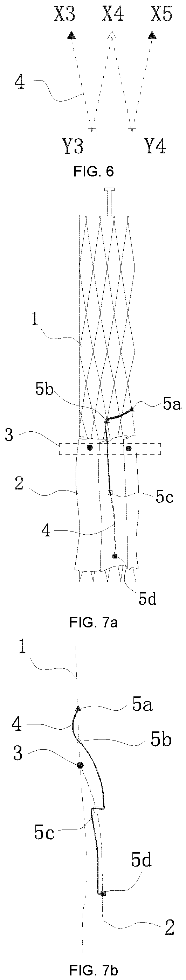

Referring to Fig. 6, in this embodiment, the outer periphery of the stent is provided with a flexible skirt. Before the stent is released, the skirt is in an unfolded configuration, which is axially unfolded and surrounding the outer periphery of the stent before release. After the stent is released, the skirt is folded and stacked along the axial direction of the stent after release into a stacked configuration and forms an annular peripheral leakage occluder. Also provided is a pulling string 4 that is configured to drive the skirt into a stacked configuration. The pulling string 4 is cooperative with the radial deforming of the stent during release.

In this embodiment, the motions of adjacent pulling units are in cooperative to each other during deformation. Two pulling units are taken as an example in the figure, and the same principle is applicable to more than two pulling units.

Within the first pulling unit: an action portion X3 is fixed on the skirt; an action portion X4 movably threads through the stent, which may thread through the inherent space of the stent, or limiting rings or holes additionally provided to guide the threading direction of the pulling string and a force exerting portion Y3 movably threads through the skirt.

Within the second pulling unit: an action portion X4, as a common action portion X4 shared with the first pulling unit, movably threads through the stent; an action portion X5 is fixed on the stent;; and a force exerting portion Y4 movably threads through the skirt.

This portion of the pulling string 4 is generally W-shaped, and the overall length of the W-shaped pulling string is fixed. When the stent is being released, the three action portions move away from each other. As the common action portion X4 movably threads through the stent the portions of the pulling string corresponding to the two pulling units may be pulled against each other and possibly with a displacement there between. As a result, the two pulling units influence and interact each other when deforming. Therefore, the two pulling units move in cooperation with each other. The same principle is applicable to more than two pulling units.

Fifth embodiment

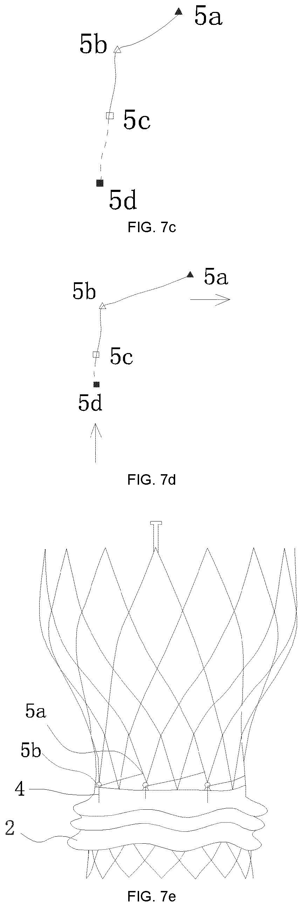

Referring to Figs. 7a to 7e, in this embodiment, the outer periphery of the stent is provided with a flexible skirt. Before the stent is released, the skirt is in an unfolded configuration, which is axially unfolded and surrounding the outer periphery of the stent before release. After the stent is released, the skirt is folded and stacked along the axial direction of the stent after release into a stacked configuration and forms an annular peripheral leakage occluder. Also provided is a pulling string 4 that is configured to drive the skirt to transform into a stacked configuration. The pulling string 4 is cooperative with radial deforming of the stent during release.

The top edge of the skirt 2 is provided with a fixing band 3, which is discontinuously fixed around the outer periphery of the stent 1. The lower portion of the skirt 2 floats around the stent 1. The skirt 2 has a circumferentially folded structure before the stent 1 is released, which makes it possible for the skirt to conform the outer periphery of the stent 1 smoothly. After the stent is released, the folded structure is unfolded. During release in the human body, the bottom edge of the skirt 2 is released first, and the top edge of the skirt 2 is released later.

One end of the pulling string 4 is a driving portion which is connected with the fixed point 5a of the stent, and then the pulling string movably threads through the turning point 5b of the stent 1 and the threading hole 5c of the skirt 2. The other end of the driving portion is a force exerting portion 5d fixed to the bottom edge of the skirt. The circumferential span between the fixed point 5a and the turning point 5b of the stent changes after the stent is released with respect to that before the stent being released. There are 2 to 8 pulling strings 4 arranged in the axial direction, however only one pulling string is shown for clarity and avoiding interference.

In order to provide the turning point 5b, a guiding hole may be provided on the stent or an inherent space of the stent itself may be utilized so as to define the position of the turning point. The inner wall of the guiding hole acts as the turning point 5b. The pulling string threads through the guiding hole in such a manner that the movement of the pulling string causes axial pulling thereof when one end of the pulling string moves along with the stent.

There may be a plurality of threading holes 5c provided in the skirt and arranged in the axial direction, and the pulling string 4 threads into and out of the skirt through the plurality of threading holes 5c until the bottom edge of the skirt 2.

The stent 1 is in a meshed structure having a plurality of cells. The circumferential distance between the fixed point 5a and turning point 5b substantially spans two cells. The fixed point 5a and turning point 5b are adjacent to each other before the stent is released, and the distance there between increases after the stent is released. The increased distance corresponds to the axially pulling distance of the pulling string 4 which drives the force exerting portion 5d to move axially and thus drives the skirt 2 to be stacked.

The axial positions of the fixed point 5a and turning point 5b may be aligned or arranged in a staggered manner. As shown in the figures, the axial position of the fixed point 5a is higher than that of the guiding hole 5b, avoiding interference there between. Both the fixed point 5a and the turning point 5b are located in the area of the stent that is not surrounded by the skirt, which facilitates to reduce the diameter of the stent being compressed and loaded and thus improves the compliance for delivery in the human body.

Sixth embodiment

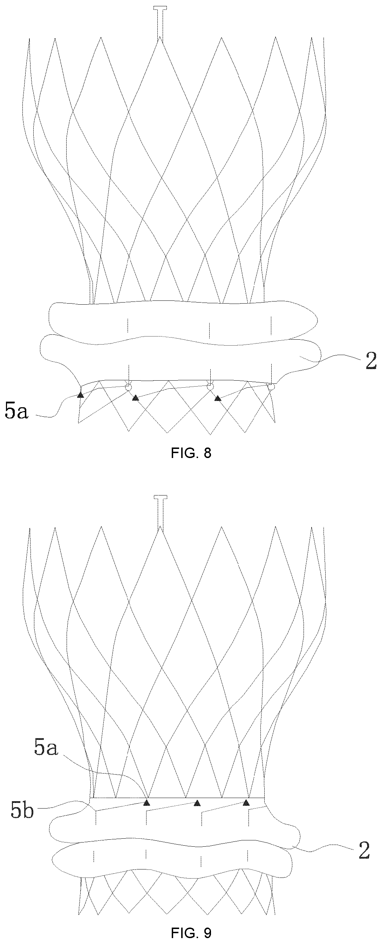

Referring to Fig. 8, compared to the fifth embodiment, the bottom edge of the skirt 2 in this embodiment is fixed, and accordingly the fixed point 5a is located below the skirt. In other words, during the stack of the skirt 2, the top edge of the skirt 2 moves towards the bottom edge thereof.

Seventh embodiment

Referring to Fig. 9, compared to the fifth embodiment, both the fixed point 5a and the turning point 5b in this embodiment are located within the area of the stent surrounded by the skirt. The top end of the pulling string with the skirt is fixed to the stent, and thus the pulling string acts as a fixing band simultaneously.

Eighth embodiment

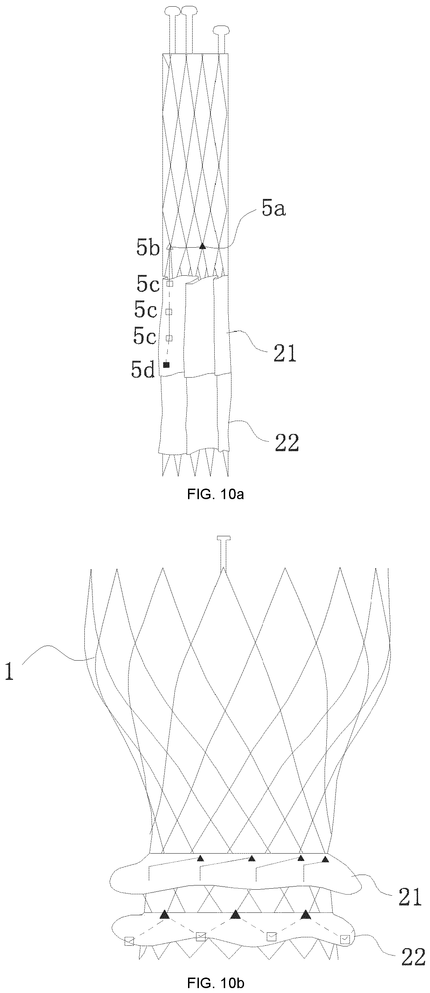

Referring to Fig. 10a, compared to the fifth embodiment, there are two skirts, that is, the upper skirt 21 and the lower skirt 22, in this embodiment. Respective pulling strings are provided for the two skirts, which are arranged in the same manner. The top edges of the skirts are fixed on the stent. Only one of the pulling strings for the upper skirt 21 is illustrated in the figures.

Both the fixed point 5a and the turning point 5b are located in the area of the stent that is not surrounded by the skirt. The pulling string threads into and out of the skirt through three threading holes 5c which are arranged axially one after another. The bottom end of the pulling string, i.e. the force exerting portion 5d, is fixed at the bottom edge of the skirt.

Referring to Fig. 10b, in another development, the respective pulling strings for the upper skirt 21 and the lower skirt 22 are arranged in different manners. The pulling string for the upper skirt 21 is arranged in the same manner as that shown in Fig. 10a, and thus the skirt is directly pulled in the axial direction. However, the pulling string for the lower skirt 22 is arranged in the same manner as that described in the first embodiment, that is, the pulling string is connected between the stent and the skirt in an undulation manner to pull the skirt.

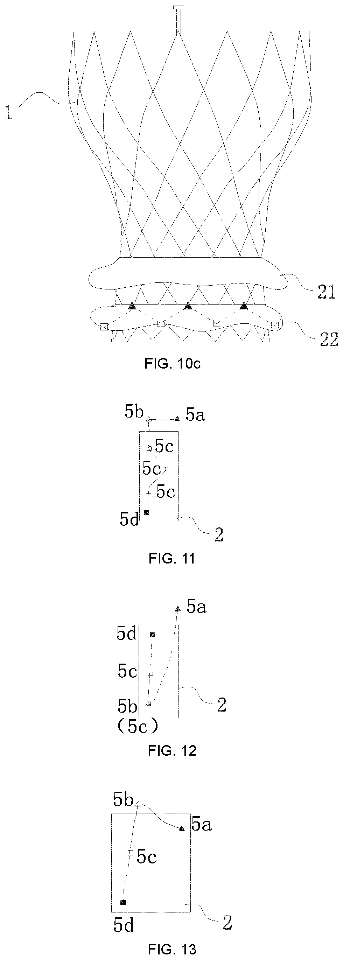

Referring to Fig. 10c, in another development, the upper skirt 21 and the lower skirt 22 adopt different driving mechanisms. The upper skirt 21 is provided with no pulling string, and is tightly mounted around the outer periphery of the stent due to its own elasticity thereof and is self-stacked during the release of the stent. In order to limit the position of the skirt, the top edge of the upper skirt 21 is fixed to the stent, and the lower portion thereof serves as a floating section. The lower skirt 22 adopts the same driving mechanism as that described in the first embodiment, that is, a pulling string is provided which is connected between the stent and the skirt in an undulation manner to pull the skirt.

Ninth embodiment

Referring to Fig. 11, compared to the eighth embodiment, one single skirt 2 is provided in this embodiment. The top edge of the skirt is fixed on the stent, with only one of the pulling strings is shown in the figure. Both the fixed point 5a and the turning point 5b are located in the area of the stent that is not surrounded by the skirt. The pulling string threads into and out of the skirt via three threading holes 5c, which are arranged axially one after another. One of the threading holes 5c is offset from the others in the axial direction such that the skirt being pulled would be slightly twisted circumferentially, improving the fullness of the profile of the peripheral leakage occluder. The bottom end of the pulling string, i.e. the force exerting portion 5d, is fixed at the bottom edge of the skirt.

Tenth embodiment

Referring to Fig. 12, compared to the ninth embodiment, the bottom edge of the skirt in this embodiment is fixed on the stent, and the upper portion thereof serve as a floating section. The fixed point 5a is located above the skirt 2, while the turning point 5b is located within the area surrounded by the skirt and adjacent to the bottom edge of the skirt. After the pulling string passing through two threading holes 5c, the force exerting portion 5d of the pulling string is fixed on the top edge of the skirt. The top edge of the skirt being pulled moves towards the bottom edge thereof.

Eleventh embodiment

Referring to Fig. 13, compared to the ninth embodiment, the fixed point 5a in this embodiment is located within the area of the stent surrounded by the skirt 2, and the turning point 5b is located above the skirt. After passing through a threading hole 5c, the force exerting portion 5d of the pulling string is fixed on the top edge of the skirt. The bottom edge of the skirt being pulled moves towards the top edge thereof.

Twelfth embodiment

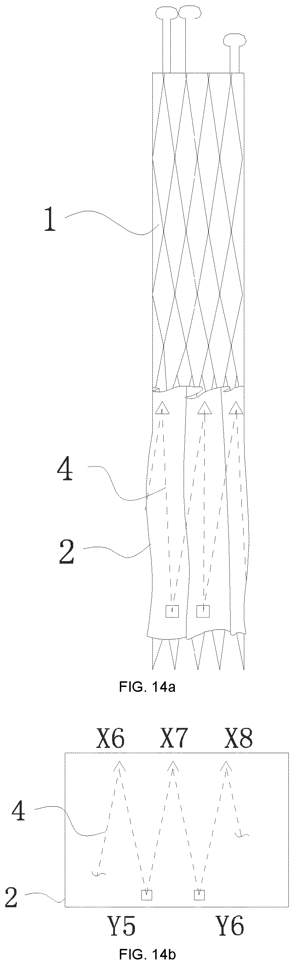

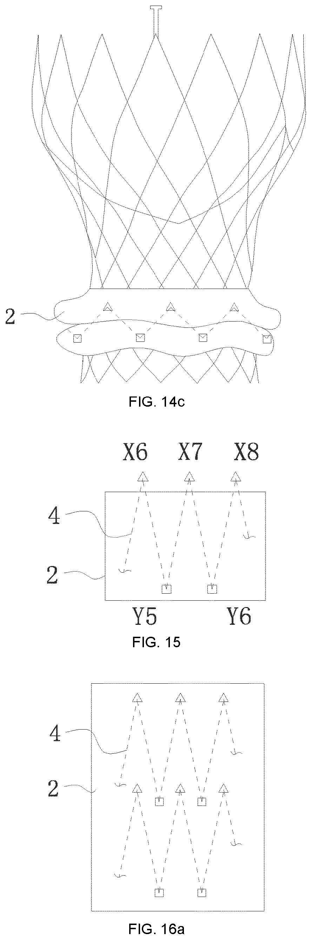

Referring to Figs. 14a to 14c, in this embodiment, the outer periphery of the stent is provided with a flexible skirt. Before the stent is released, the skirt is in an unfolded configuration, which is axially unfolded and surrounding the outer periphery of the stent before release. After the stent is released, the skirt is folded and stacked along the axial direction of the stent after release into a stacked configuration and forms an annular peripheral leakage occluder. Also provided is a pulling string 4 that is configured to drive the skirt to transform into a stacked configuration. The pulling string 4 is cooperative with radial deforming of the stent during release.

The top edge of the skirt 2 is fixed to the stent 1 by threading the pulling string 4 through the stent and skirt, and the lower portion of the skirt 2 floats around the stent 1. The skirt 2 has a circumferentially folded structure, which makes it possible for the skirt to conform the outer periphery of the stent 1 smoothly. After the stent is released, the folded structure is unfolded. During release in the human body, the bottom edge of the skirt 2 is released first, and the top edge of the skirt 2 is released later.

Hollow triangular boxes denote that the portions of the pulling string there movably thread through the stent. If the corresponding areas of the stent in the present embodiment are surrounded by the skirt, the pulling string threads through the corresponding portions of the skirt for the axially positioning the skirt relative to the stent. Hollow rectangular boxes denote that the portions of the pulling string there movably thread through the skirt without threading through the stent.

In this embodiment, the pulling string generally threads between the skirt and the stent in a circumferential direction, which undulates in the axial direction of the stent while extending along the circumferential direction, thus forming an undulation structure with peaks and valleys. In order to improve the cooperation effect, the peaks of the pulling string movably thread through the stent, and the valleys movably thread through the skirt. Within one wave of the undulation structure, the axial length of the wave is greater than the circumferential length thereof , which is able to improve the pulling performance, and results in a greater axial displacement thereof under a certain radial deformation of the stent.

Within a periodic portion of the pulling string 4 as shown in the figure, peak X6, valley Y5, peak X7, valley Y6, peak X8 are distributed in the circumferential direction one another while undulating in the axial direction. In the unfolded configuration, the peaks are in the same level in the axial position, and the valleys are in the same level in the axial position. Alternatively, the peaks (or the valleys) may also be offset from each other.

During the release of the stent, driven by the deformed stent, the peaks of the pulling string move away from each other in the circumferential direction and thus drive the valleys to lift upwards. Since the pulling string movably threads through the stent the and skirt, the periodic portions thereof are configured for driving and operating in cooperation with one another, thereby generally moving the pulling string.

Thirteenth embodiment

Referring to Fig.15, within a periodic portion of the pulling string 4 of this embodiment, peak X6, valley Y5, peak X7, valley Y6, and peak X8 are distributed in the circumferential direction one another while undulating in the axial direction. Different from the twelfth embodiment, the peaks here are located at the outside of the skirt 2. In other words, the corresponding portions of the stent are not covered by the skirt. In this situation, a fixing band may be provided at the top edge of the skirt to further limit the axial position of the top edge of the skirt relative to the stent.

Fourteenth embodiment

Referring to Fig. 16a, this embodiment differs from the twelfth embodiment in that two sets of pulling strings 4 are provided here, one above another in an axial direction, which are configured to pull the corresponding portions of the skirt 2 at different stages during releasing of the stent, respectively. The peaks of the lower pulling string are located higher than the valleys of the upper pulling string. Each pulling string moves together in the circumferential direction to pull the skirt.

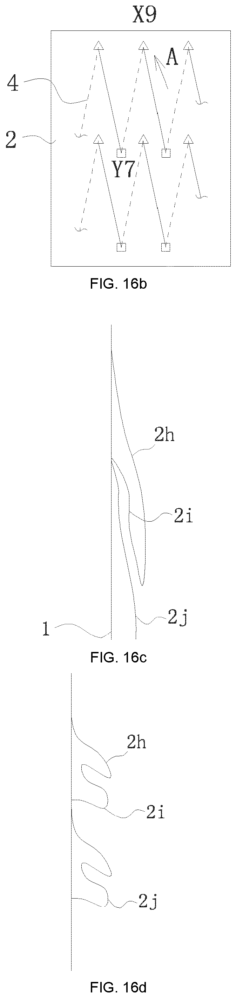

Referring to Fig. 16b, in order to further show the threading of the pulling string through the skirt, the dashed lines in the figure indicate the portions of the pulling string located beneath the skirt, and the solid lines indicate the portions of the pulling string located above the skirt. The pulling string threads into or out of the stent depending on the location relationship between the pulling string and the skirt, i.e., beneath or above the skirt. For example, when the pulling string threads through the skirt at peaks X9 along the direction of arrow A, it may be followed by threading through the stent and entering into the interior of the stent subsequently and directly. However, it is required for the pulling string to return to the position between the stent and the skirt soon, and extend between the stent and the skirt to the valleys Y7 such that the corresponding portion of skirt is able to float.

Referring to Figs. 16c and 16d, the skirt 2 includes two sections corresponding to the two pulling strings respectively although the skirt 2 is an integrated structure as one single piece. Skirt sections 2h and 2i are folded axially at the outer periphery of the stent 1 to float. The skirt section 2i extends downwards (i.e. towards the end of the stent) after being fixed with the stent 1 to form the skirt section 2j which itself serves as a floating section and the free end thereof is a floating edge.

The connection of the skirt sections 2h and 2i is located below the top edge of skirt section 2j, so that the peaks and valleys of the two pulling strings may be distributed in a staggered manner. In the stacked configuration, the skirt sections 2h and 2i are stacked subjected to the pulling of the upper pulling string with the skirt section 2j unaffected, and the skirt section 2j is stacked independently subjected to the pulling of the lower pulling string.

Fifteenth embodiment

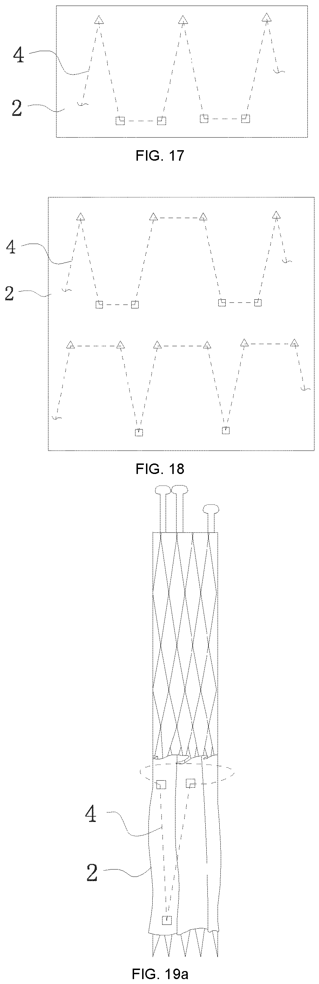

Referring to Fig. 17, this embodiment differs from the twelfth embodiment in the wave shape of the undulation structure of the pulling string 4. In this embodiment, the pulling string follows a course having a wave shape with sharp peaks and flat valleys. The portions of the stent where the pulling string movably threads through are surrounded by the skirt 2.

Sixteenth embodiment

Referring to Fig. 18, this embodiment differs from the fourteenth embodiment in the wave shape of the undulation structure of the pulling string 4. The wave shapes of the two pulling strings are different. The upper pulling string follows the course having a wave shape with flat peaks and flat valleys; and the lower pulling string follows a course having a wave shape with flat peaks and sharp valleys. The portions of the stent where the pulling string movably threads through are surrounded by the skirt 2.

Seventeenth embodiment

Referring to Fig. 19a, this embodiment differs from the thirty-second embodiment in that the pulling string 4 here is routed around the stent for less than a perimeter of the stent to form substantially an annular driving portion. Opposite ends of the driving portion movably thread through the skirt 2 respectively, and then extend downwards and join together to form a force exerting portion after movably threading through the bottom of the skirt. The annular driving portion may thread through the stent, or may be just surrounding the outer periphery of the stent. A single force exerting portion is provided as shown in the Fig. 19a. Alternatively, more than one force exerting portions may be provided. It is also possible to provide a plurality of pulling strings 4, which pull the corresponding force exerting portions at the bottom of the skirt, respectively.

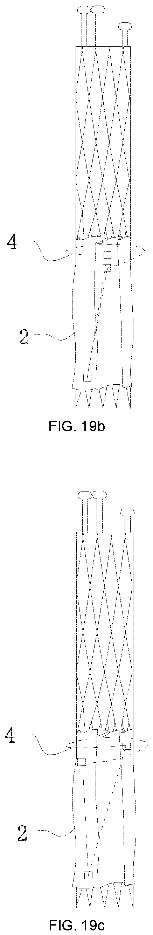

Referring to Fig. 19b, in a further development, the pulling string 4 extends further in the circumferential direction compared to that shown in Fig. 19a. In particular, the pulling string 4 in this embodiment extends for the entire perimeter of the stent and form an annular driving portion surrounding the stent.

Referring to Fig. 19c, in a further development, the pulling string 4 extends further in the circumferential direction compared to that shown in Fig. 19a. In particular, the pulling string 4 in this embodiment extends in the circumferential direction for 1.5 times of a perimeter of the stent, and forms an annular driving portion.

Eighteenth embodiment

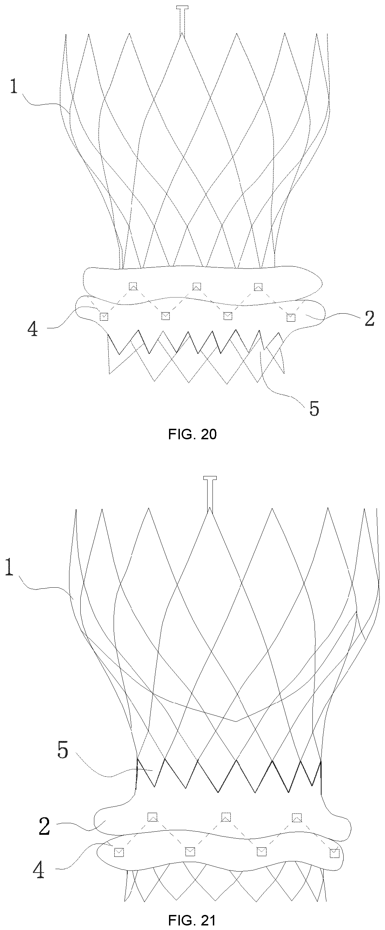

Referring to Fig. 20, in this embodiment, the outer periphery of the stent 1 is provided with a flexible skirt 2. Before the stent 1 is released, the skirt 2 is in an unfolded configuration, which is axially unfolded and surrounding the outer periphery of the stent 1 before release. After the stent 1 is released, the skirt 2 is folded and stacked along the axial direction of the stent 1 after release into a stacked configuration and forms an annular peripheral leakage occluder. Also provided is a pulling string 4 that is configured to drive the skirt 2 to transform into the stacked configuration. The pulling string 4 is cooperative with radial deforming of the stent during release. When being released in the human body, the bottom edge of the skirt 2 is released first, and the top edge of the skirt 2 is released later.

In this embodiment, the pulling string generally threads through the skirt in the circumferential direction. The pulling string undulates in the axial direction while extending in the circumferential direction, forming a wave-like configuration with peaks and valleys.

During the release of the stent, driven by the deformed stent, the peaks of the pulling string move away from each other in the circumferential direction and thus drive the valleys to lift upwards. Since the pulling string movably threads through the skirt, the periodic portions thereof are configured for driving and operating in cooperation with one another, thereby generally moving the pulling string.

The top edge of the skirt 2 is fixed on the stent. The skirt 2 is provided with a plurality of cutting areas 5 at the bottom side of the skirt, arranged in the circumferential direction. The cutting areas may reduce the radially stacking thickness of the skirt in the unfolded configuration, facilitating loading onto the stent and delivery of the stent apparatus.

The bottom edge of the skirt 2 is generally in a tooth-shaped structure extending in the circumferential direction, with a cutting area formed between two adjacent teeth. In this embodiment, the teeth are triangular and evenly arranged in the circumferential direction.

Before the stent is released, the distal end of the skirt to be released first extends beyond the stent in the axial direction, and it is the tooth-shaped structure that extends beyond the stent.

Nineteenth embodiment

Referring to Fig. 21, this embodiment differs from the eighteenth embodiment in that the top edge of the skirt 2 here is fixed on the stent and has a plurality of cutting areas 5. The top edge of the skirt 2 corresponds to the edges of the grids of the stent, and is continuously stitched on the stent along the edges of the grids.

Twentieth embodiment

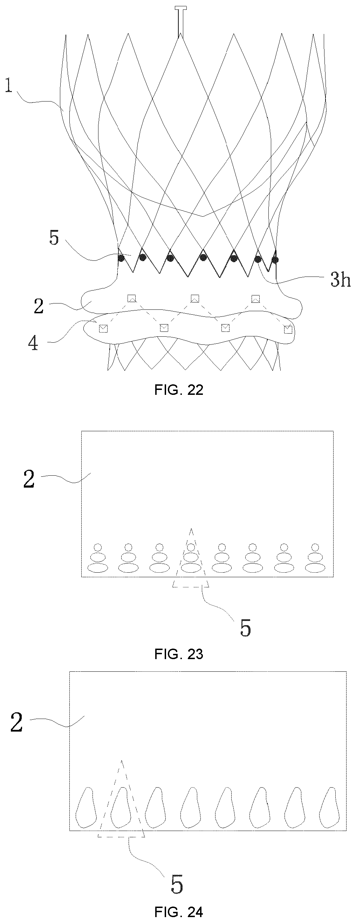

Referring to Fig. 22, the top edge of the skirt 2 has a tooth-shaped structure with triangular teeth. This embodiment differs from the nineteenth embodiment in that the top tips of the triangular teeth at the top edge of the skirt 2 are fixed on the stent with a plurality of fixed points 3h spaced from each other.

Twenty-first embodiment

Referring to Fig. 23, this embodiment differs from the eighteenth embodiment in that each of the cutting areas 5 here includes a plurality of through holes arranged along the axial direction of the stent, with solid materials arranged between the through holes, which facilitates the overall shaping of the peripheral leakage occluder and maintenance of necessary pull at various areas.

The through hole may be in a circular or an elliptical shape. Among the through holes in a same cutting area, the closer to the corresponding side edge of the skirt, the larger size the through hole has.

Twenty-second embodiment

Referring to Fig. 24, this embodiment differs from the twenty-first embodiment in that the cutting area here includes a single through hole. The inner edge of the through hole is relatively smooth. The closer to the corresponding side edge of the skirt, the larger width the through hole has.

Twenty-third embodiment

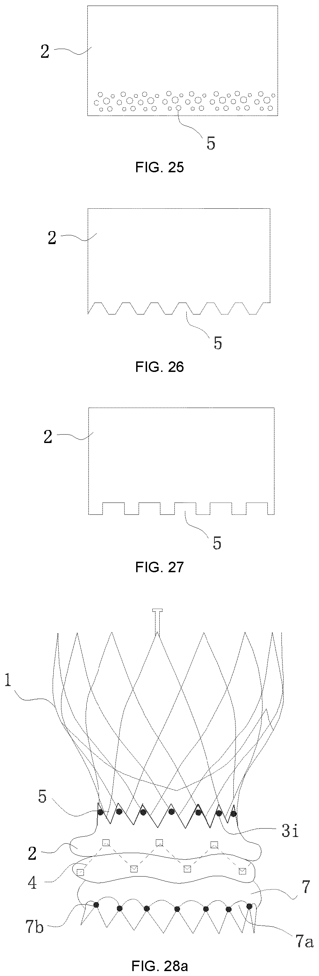

Referring to Fig. 25, this embodiment differs from the twenty-first embodiment in that there is no obvious boundary between the cutting areas 5 here, and the cutting areas 5 are provided with a plurality of through holes densely arranged.

Twenty-fourth embodiment

Referring to Fig. 26, this embodiment differs from the eighteenth embodiment in that the teeth between two adjacent cutting areas are trapezoid-shaped here.

Twenty-fifth embodiment

Referring to Fig. 27, this embodiment differs from the twenty-fourth embodiment in that the teeth between two adjacent cutting areas are rectangle-shaped here.

Twenty-sixth embodiment

Referring to Fig. 28a, in this embodiment, the outer periphery of the stent 1 is provided with a flexible skirt 2. Before the stent 1 is released, the skirt 2 is in an unfolded configuration, which is axially unfolded and surrounding the outer periphery of the stent 1 before release. After the stent 1 is released, the skirt 2 is folded and stacked along the axial direction of the stent 1 after release into a stacked configuration and forms an annular peripheral leakage occluder. Also provided is a pulling string 4 that is configured to drive the skirt 2 to transform into the stacked configuration. The pulling string 4 is cooperative with radial deforming of the stent during release. The top edge of the skirt 2 has a tooth-shaped structure with triangular teeth, and the top tips of the triangular teeth are fixed on the stent with a plurality of fixed points 3h spaced from each other. The lower portion of the skirt 2 is a floating section. When being released in the human body, the bottom edge of the skirt 2 is released first, and the top edge of the skirt 2 is released later.

In this embodiment, the pulling string generally threads through the skirt in the circumferential direction. The pulling string undulates in the axial direction while extending in the circumferential direction, forming a wave-like configuration with peaks and valleys. The peaks are located at the top edge of the skirt 2. Both peaks and valleys movably thread through the skirt 2. However, the pulling string does not thread through the stent. During the release of the stent, driven by the deformed stent, the peaks move away from each other in the circumferential direction and thus drive the valleys to lift upwards.

The normal blood inflow side of the stent 1, i.e., the bottom end thereof, is surrounded by a pushing pocket 7 for facilitating the skirt 2 transforming into the stacked configuration. The pushing pocket 7 has inlets for allowing blood to flow in, such that the pushing pocket 7 is self-expandable to push the skirt. The pushing pocket is made of flexible material, which may be the same material as the skirt. The pushing pocket has a double-layer structure, so it allows to be filled with blood and expanded radially.

One side of the pushing pocket 7 is closed, and the other side is provided with a plurality of stitching portions 7b fixed to the stent 1. Adjacent stitching portions are spaced from each other, with an inlet defined there between. Therefore, a plurality of inlets 7a for allowing back-flow blood to flow therein are provided in this embodiment, which are evenly arranged along the circumferential direction. In an unfolded configuration of the skirt, the bottom edge of the skirt extends beyond the pushing pocket, so that the bottom edge of the skirt covers the pushing pocket 7 fully or partially.

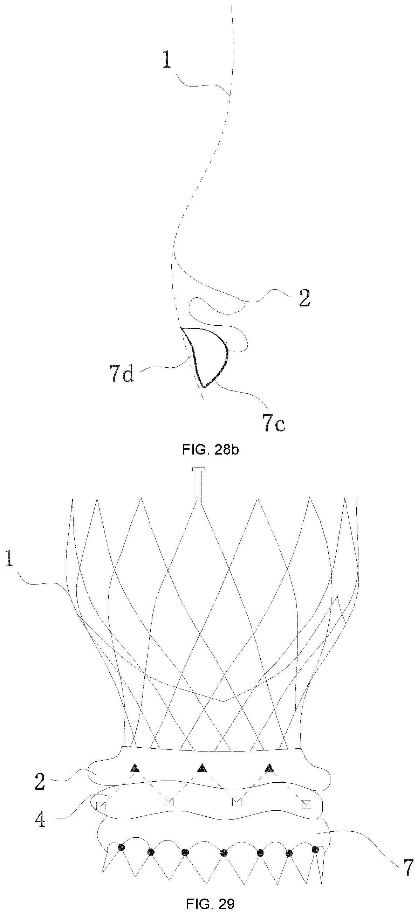

Referring to Fig. 28b, which schematically illustrates a cross section of the pushing pocket 7. In this embodiment, the dashed line indicates the stent 1. The pushing pocket 7 is separately provided with respect to the material of the skirt 2. The pushing pocket 7 has a double-layer structure, including an inner layer 7d and an outer layer 7c. The two layers 7d and 7c may be formed as one single piece. The two layers are integrally connected with a bend at the bottom. In this embodiment, the inner layer 7d and the outer layer 7c are separately formed, and are connected and sealed by stitching the tops together.

Twenty-seventh embodiment

Referring to Fig. 29, this embodiment differs from the twenty-sixth embodiment in that the skirt 2 does not have a tooth-shaped structure at the top edge.

Twenty-eighth embodiment

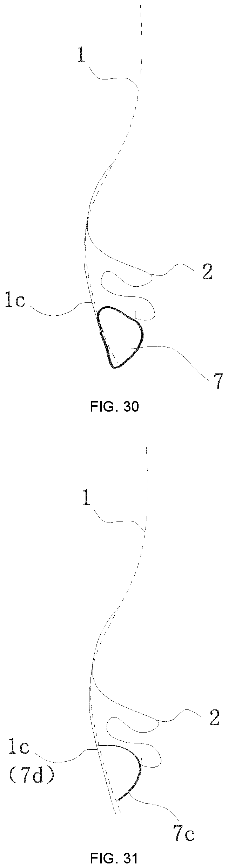

Referring to Fig. 30, the dashed line indicates the stent 1. Different from the twenty-sixth embodiment, in this embodiment, an inner coverage membrane 1c is provided at the inner wall of the stent 1, the material of which is turned outwards and folded to form the pushing pocket 7.

Twenty-ninth embodiment

Referring to Fig. 31, the dashed line indicates the stent 1. Different from the twenty-sixth embodiment, in this embodiment, an inner coverage membrane 1c is provided at the inner wall of the stent 1, which simultaneously serves as the inner layer 7d of the pushing pocket 7. The outer layer 7c of the pushing pocket 7 is separately provided. A top edge of the outer layer 7c is stitched and sealed to the corresponding portion of the inner coverage membrane 1c.

Thirtieth embodiment

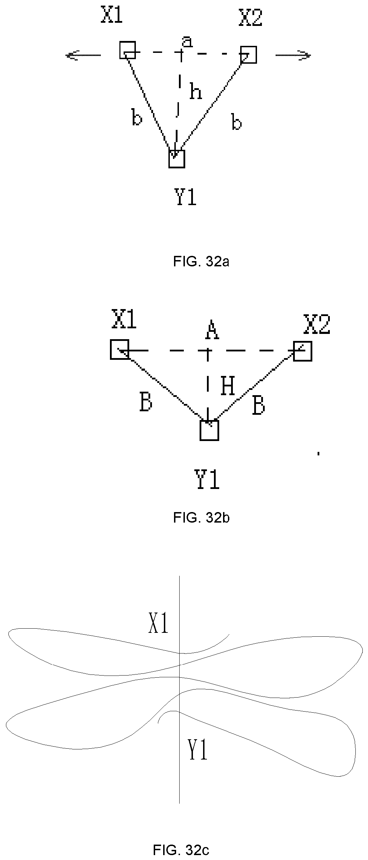

As shown in Figs. 32a and 32b, in this embodiment, a single pulling unit is taken as an example for illustration. Assuming that the length of the pulling string is l, the distance between the first acting portion X1 and the second acting portion X2 before being pulled is a, and the distance between the force exerting portion Y1 and a line connecting the first acting portion X1 and the second acting portion X2 before being pulled is h.

Before being pulled, the distance between the first acting portion X1 and the force exerting portion Y1 is b.

Before being pulled, the distance between the second acting portion X2 and the force exerting portion Y1 is b.

Before being pulled, the overall length of the pulling string of the pulling unit in the Fig. 32a is 2b, where 2b = l.

After being pulled, the distance between the first acting portion X1 and the second acting portion X2 is A, and the distance between the force exerting portion Y1 and a line connecting the first acting portion X1 and the second acting portion X2 is H.

After being pulled, the distance between the first acting portion X1 and the force exerting portion Y1 is B.

After being pulled, the distance between the second acting portion X2 and the force exerting portion Y1 is B.

After being pulled, the overall length of the pulling string of the pulling unit in the Fig. 32b is 2B, where 2B = l.

After being pulled, the circumferential distance variation of the pulling string is: $\Delta a$ = A-a. The axial distance variation of the force exerting portion Y1 before and after being pulled is:

$$\Delta h = h - H = \sqrt{b^2 - \left(\frac{a}{2}\right)^2} - \sqrt{B^2 - \left(\frac{A}{2}\right)^2}$$

$$= \sqrt{\left(\frac{l}{2}\right)^2 - \left(\frac{a}{2}\right)^2} - \sqrt{\left(\frac{l}{2}\right)^2 - \left(\frac{A}{2}\right)^2}$$

$$= \frac{\sqrt{l^2 - a^2} - \sqrt{l^2 - A^2}}{2}$$

$$= \frac{\sqrt{l^2 - a^2} - \sqrt{l^2 - (a + \Delta a)^2}}{2}$$

It can be seen that the axially pulling length of the pulling unit is closely related to the circumferential distance variation between the first acting portion and the second acting portion. In most cases, the circumferential distance variation of the pulling string from the unfolded configuration to the stacked configuration is less than a perimeter of the stent.

The thickness of the peripheral leakage occluder after being folded is related to the number of folds. Referring to Fig. 32c, assuming that there are three threading holes between the first acting portion X1 and the force exerting portion Y1, if the pulling string is tensioned, five layers of folds will be formed.

In a further development, the stent apparatus is a balloon expandable stent, with the stent pre-compressed onto the balloon. During release, the balloon is expanded by injected saline solution to expand the stent. With reference to Figs. 25a and 25b, the skirt is stitched on the stent, and when the whole stent is expanded, the distance between the first action portion X1 and the second action portion X2 increases, which pulls the force application portion Y1 to move axially.

Thirty-first embodiment

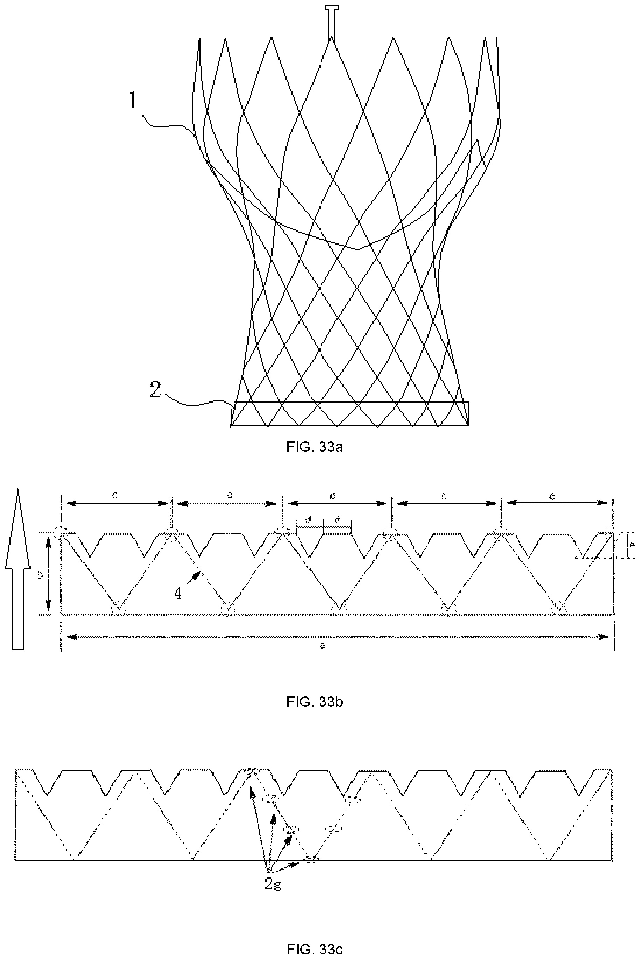

Referring to Figs. 33a to 33c, in this embodiment, the outer periphery of the stent 1 is provided with a flexible skirt 2. Before the stent 1 is released, the skirt 2 is in an unfolded configuration, which is axially unfolded and surrounding the outer periphery of the stent 1 before release. After the stent 1 is released, the skirt 2 is folded and stacked along the axial direction of the stent 1 after release into a stacked configuration and forms an annular peripheral leakage occluder. Also provided is a pulling string 4 that is configured to drive the skirt 2 to transform into the stacked configuration. The pulling string 4 is cooperative with radial deforming of the stent 1 during release. When being released in the human body, the bottom edge of the skirt 2 is released first, and the top edge of the skirt 2 is released later.

The Fig. 33a only illustrates the position of the skirt 2, in which the skirt 2 is substantially located at one axial end of the stent 1 which is adjacent to the blood inflow side (the direction of the hollow arrow in the Fig. 33b indicates the normal blood flow direction when the stent is in use). The stent 1 has a meshed structure, for example, having a plurality of grids as shown in the figures. The axial length of the skirt in the unfolded configuration is half of the axial length of a grid.

The top edge of the skirt 2 is fixed on the stent, and the skirt 2 is provided with a plurality of cutting areas 5 at the axial top side thereof, arranged in the circumferential direction. The cutting areas may reduce the radially stacking thickness of the skirt in the unfolded configuration, facilitating the load and delivery. The top edge of the skirt 2 is generally in a tooth-shaped structure extending in the circumferential direction, with a cutting area formed between two adjacent teeth, and the teeth are triangular or trapezoidal and evenly arranged in the circumferential direction.

In the case that the top edge of the skirt 2 is fixed on the stent, stitching portions may be provided only at a middle portion of the top edge of the tooth-shaped structure, with the stitching portions circumferentially spaced from each other. Developing points are provided on the stent at the stitching portions.

In this embodiment, the pulling string generally threads through the skirt in the circumferential direction. The pulling string undulates in the axial direction while extending in the circumferential direction, forming a wave-like configuration with peaks and valleys.

During the release of the stent, driven by the deformed stent, the peaks of the pulling string move away from each other in the circumferential direction and thus drive the valleys to lift upwards. Since the pulling string movably threads through the skirt, the periodic portions thereof are configured for driving and operating in cooperation with one another, thereby generally moving the pulling string.

The portions indicated by broken line circles in the figures may serve as the threading holes 2g. The pulling string 4 only threads through the skirt, into and out of the threading holes.

In a further development, the pulling string may thread through the stent at the uppermost threading holes in the figure, which also serves as the stitching portions and thus maintains the axial position of the skirt relative to the stent.

In an unfolded configuration of the skirt, the skirt has a length a substantially equaling to the perimeter of a corresponding portion of the stent, and the skirt has a width b which is half of the height of a grid of the stent. The length of the skirt is evenly divided into five parts, with the length of each part being c. The length of each part is equal to the length of two complete teeth. In other words, there are 10 teeth in total. Each tooth has a length d (the upper side of the trapezoid) which is equal to a distance between two adjacent teeth d. The width of the skirt is evenly divided into four parts, with the length of each part being e. The height of each tooth is e.

In the stacked configuration, each pulling unit may be folded for three times (fold is generated between two adjacent threading holes in the axial direction) to form a four-layer stack provided that a plurality of threading holes 2g are provided. By providing a plurality of threading holes, the position limiting between the pulling string and the skirt can be increased, so that the skirt can be stacked more uniformly when the skirt is pulled.

Specifically, taking a specific valve as an example, the skirt has a length a, where a = 95mm $\pm$ 0.2mm, and the skirt has a width b, where b = 8.80mm $\pm$ 0.2mm.

the length of the skirt may be divided into five parts, with the length of each part being c, where c = 95/5 = 19mm $\pm$ 0.2mm.

The tooth has a length d, and the distance between two adjacent teeth is also d, where d = 19/4 = 4.75mm $\pm$ 0.2mm.

Each tooth has a height e, where e = 8.81 / 4 = 2.2mm $\pm$ 0.2mm.

Thirty-second embodiment

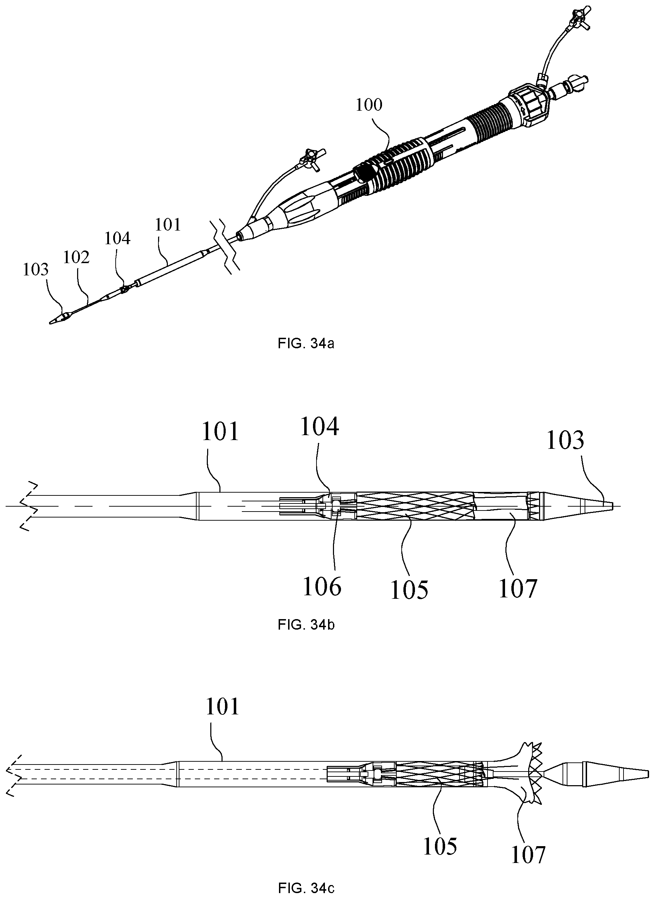

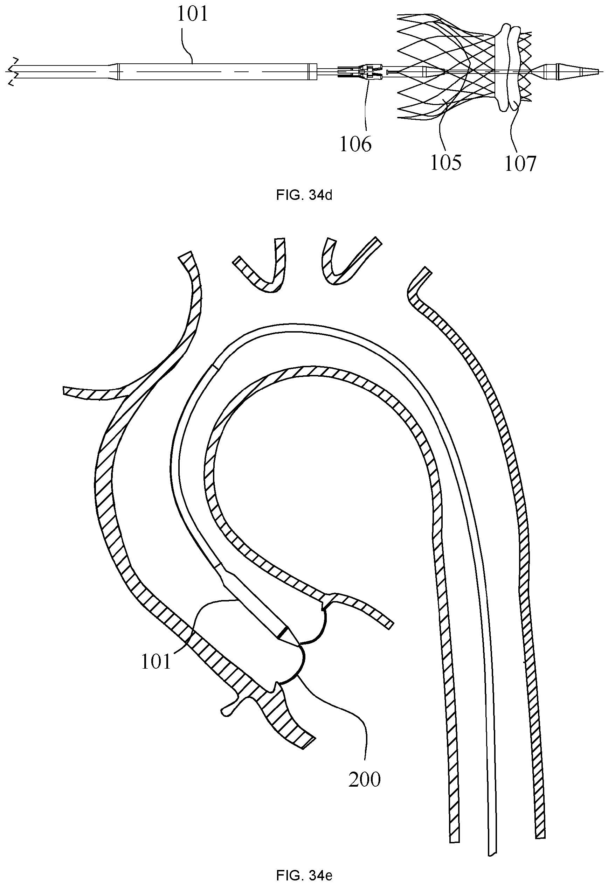

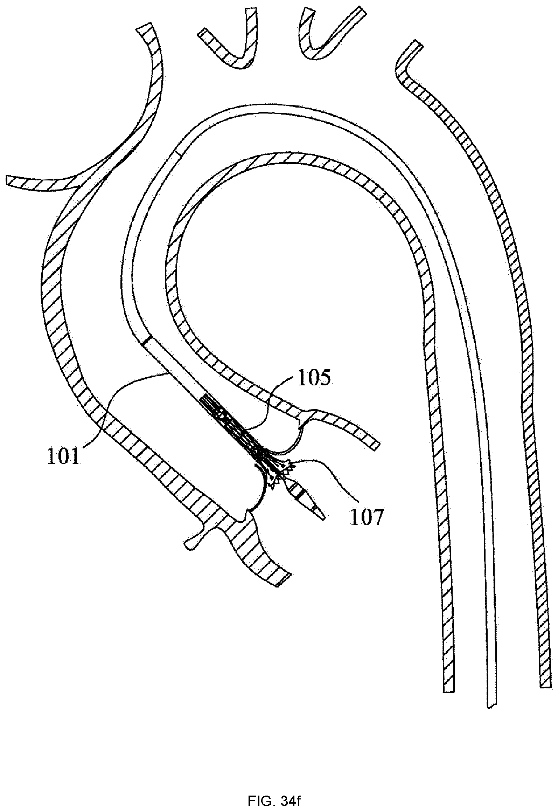

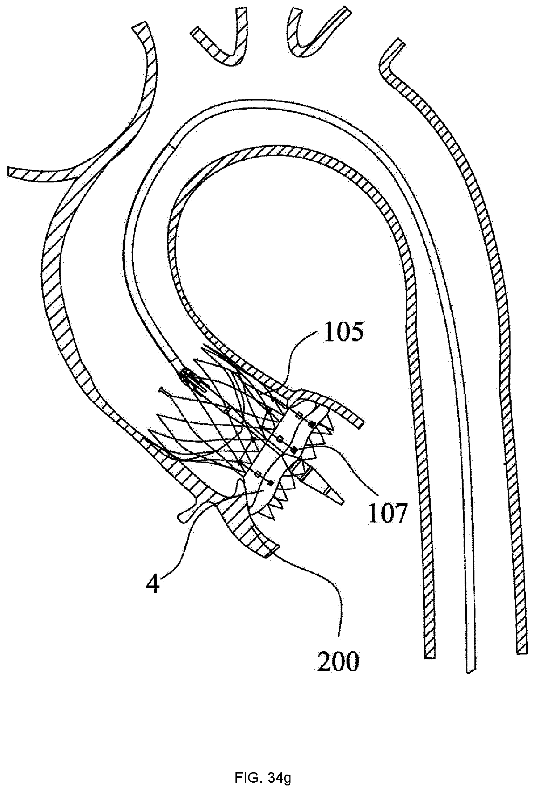

Referring to Figs. 34a to 34g, a delivery system is provided in this embodiment, which includes a handle 100, a core shaft 102 connected to the handle 100, and an sheath 101 slidingly surrounding the outer periphery of the core shaft 102. The sheath 101 is able to slide axially relative to the core shaft 102 under controlling of the handle 100.

The distal end of the core shaft 102 is provided with a guide head 103 and a mounting head 104 adjacent to the guide head 103. The mounting head 104 is provided with a plurality of slots or protrusions for connecting with an implanting instrument. The implanting instrument may be, for example, the stent 105 with the skirt 107 in the above embodiments. The proximal end of the stent 105 is provided with a fixing ear 106 for engaging with the mounting head 104. In a loaded state, the stent 105 is radially compressed, located between the guide head 103 and the mounting head 104, and constrained by the sheath 101. Correspondingly, the skirt is in an unfolded configuration, and arranged around the outer periphery of the stent 105.

After the stent 105 is delivered to a desired position in the human body such as a lesion site near the aortic valve 200 by the delivery system, the sheath 101 is withdrawn relative to the core shaft 102, and the distal end of the stent 105 is first released, and is gradually exposed and begins to expand radially. Under the action of the stent 105 itself or in combination with the pulling string, the skirt 107 begins to be axially pulled and stacked. After the stent 105 is completely released, the skirt 107 transitions into a stacked configuration and forms a peripheral leakage occluder, further preventing the regurgitation at the aortic valve 200.

The related principles and synergistic effects may be referred to the related description of the summary of the invention.

The above disclosure is only specific embodiments of the present invention, but the present invention is not limited thereto. Those skilled in the art may make various modifications and variations to the present invention without departing from the scope of the present invention. In addition, although some specific terms are used in this specification, these terms are just for convenience of illustration and do not constitute any special limitation to the present invention.

**Claims**

1. A stent apparatus with skirt for preventing peripheral leakage, comprising a stent (1) which has a first configuration before being released and a second configuration after being released that is different from the first configuration, the stent apparatus is further provided with a flexible skirt (2), wherein the flexible skirt (2) has:

   a stacked configuration, in which the skirt (2) is folded and stacked in the axial direction of the stent (1) after being released and forms an annular peripheral leakage occluder;
   wherein at least a portion of the skirt is fixed on the stent, and the stent apparatus for preventing peripheral leakage is further provided with a pulling string (4) for driving the skirt (2) to transform into the stacked configuration, the pulling string (4) comprises a driving portion (4a) and a force exerting portion (4b), wherein the driving portion (4a) operates in cooperation with the stent (1) during release, and the force exerting portion (4b) is connected with the skirt (2) and is pulled by the driving portion (4a) during release of the stent (1) to obtain an axial displacement thereof relative to the stent (1) so as to pull the skirt (2) to be stacked;

**characterized in**

**that** the flexible skirt (2) further has an unfolded configuration, in which the skirt (2) axially unfolded and surrounds an outer periphery of the stent (1) before being release, and neither of two ends of the skirt (2) in the unfolded configuration in an axial direction of the stent (1) extends beyond corresponding side of the stent (1).

2. The stent apparatus with skirt for preventing peripheral leakage according to claim 1, wherein one axial side edge of the stent (1) has a sharp angled structure, and before the stent (1) is released, a corresponding side of the skirt (2) does not extend axially beyond tips of the sharp angled structure.

3. The stent apparatus with skirt for preventing peripheral leakage according to claim 1, wherein before the stent (1) is released, the skirt (2) in the unfolded configuration has a folded structure in a circumferential direction.

4. The stent apparatus with skirt for preventing peripheral leakage according to claim 1, wherein one end of the driving portion (4a) is connected with a fixed point (5a) of the stent, and the driving portion (4a) is connected with the force exerting portion (4b) after passing through a turning point (5b) of the stent (1); and a relative circumferential displacement between the fixed point (5a) and the turning point (5b) is produced after the stent (1) is released with respect to that before the stent (1) being released.

5. The stent apparatus with skirt for preventing peripheral leakage according to claim 4, wherein 2 to 8 pulling strings (4) are provided which are circumferentially arranged around the stent (1), and the stent (1) is provided with a guiding hole (5b) for defining the turning point (5b).

6. The stent apparatus with skirt for preventing peripheral leakage according to claim 5, wherein the guiding hole (5b) is provided by at least one of the following manners: by an opening in the stent (1), by an inherent space formed in the stent (1), or by a ring additionally provided and attached to the stent (1).

7. The stent apparatus with skirt for preventing peripheral leakage according to claim 4, wherein at least one section of the skirt in the axial direction is configured as a floating section around the outer periphery of the stent, and the floating section has a stacked configuration resulted from the pull of the pulling string (4); one or more threading holes (5c) are provided on the floating section in an axial direction, and the pulling string (4) threads into and out of the floating section through the one or more threading holes (5c) after passing through the turning point (5b).

8. The stent apparatus with skirt for preventing peripheral leakage according to claim 7, wherein each pulling string (4) threads through 1 to 6 threading holes (5c), the circumferential positions of which are aligned with each other or arranged in a staggered manner.

9. The stent apparatus with skirt for preventing peripheral leakage according to claim 7, wherein at least one threading hole (5c) is provided on a floating section adjacent to end nodes of the stent (1).

10. The stent apparatus with skirt for preventing peripheral leakage according to claim 4, wherein the stent (1) is in a cylindrical meshed structure having a plurality of cells, and a circumferential distance between the fixed point (5a) and the turning point (5b) of the stent (1) corresponding to the pulling string (4) spans 1 to 4 cells of the stent (1).

11. The stent apparatus with skirt for preventing peripheral leakage according to claim 10, wherein the axial positions of the fixed point (5a) and the turning point (5b) are aligned with each other or arranged in a staggered manner.

12. The stent apparatus with skirt for preventing peripheral leakage according to claim 7 wherein the pulling string (4) and the floating section are connected in the following manner: an end of the pulling string (4) distant from the fixed point (5a) is connected at a distal end of a floating section which is to be released first, and the turning point (5b) is located at or adjacent to a proximal end of the floating section which is to be released later, and the axial position of the fixed point (5a) is further away from the proximal end of the floating section than the turning point (5b); or an end of the pulling string (4) distant from the fixed point (5a) is connected at a proximal end of a floating section to be released later, and the turning point (5b) is located at or adjacent to a distal end of the floating section to be released first, and the axial position of the fixed point (5a) is further away from the distal end of the floating section than the turning point (5b).

13. The stent apparatus with skirt for preventing peripheral leakage according to claim 1, wherein the pulling string (4) undulates in the axial direction of the stent (1) while extending along the stent (1) in a circumferential direction and thus form a wave-like structure.

14. The stent apparatus with skirt for preventing peripheral leakage according to claim 1, wherein the pulling string (4) threads circumferentially between the skirt (2) and the stent (1).

15. A heart valve comprising a stent apparatus and valves provided in the stent apparatus, wherein the stent apparatus is the stent apparatus with skirt for preventing peripheral leakage according to any one of claims 1 to 14.


**Patentansprüche**

1. Stenteinrichtung mit Schürze zum Verhindern einer peripheren Leckage, umfassend einen Stent (1), der eine erste Konfiguration, bevor er freigegeben wird, und eine zweite Konfiguration aufweist, nachdem er freigegeben wird, die sich von der ersten Konfiguration unterscheidet, wobei die Stenteinrichtung ferner mit einer flexiblen Schürze (2) versehen ist, wobei die flexible Schürze (2) aufweist:

    eine gestapelte Konfiguration, bei der die Schürze (2) in der axialen Richtung des Stents (1) gefaltet und gestapelt ist, nachdem sie freigegeben wird, und einen ringförmigen peripheren Leckageokkluder ausbildet;
    wobei mindestens ein Abschnitt der Schürze auf dem Stent befestigt ist, und die Stenteinrichtung zum Verhindern der peripheren Leckage ferner mit einer Zugschnur (4) zum Antreiben der Schürze (2) versehen ist, um in die gestapelte Konfiguration zu transformieren, wobei die Zugschnur (4) einen Antriebsabschnitt (4a) und einen Kraft ausübenden Abschnitt (4b) umfasst, wobei der Antriebsabschnitt (4a) während einer Freigabe in Zusammenarbeit mit dem Stent (1) arbeitet, und der Kraft ausübende Abschnitt (4b) mit der Schürze (2) verbunden ist und während der Freigabe des Stents (1) durch den Antriebsabschnitt (4a) gezogen wird, um eine axiale Verschiebung davon relativ zu dem Stent (1) zu erreichen, um die Schürze (2) zu ziehen, um gestapelt zu werden;
    **dadurch gekennzeichnet, dass** die flexible Schürze (2) ferner eine entfaltete Konfiguration aufweist, in der die Schürze (2) axial entfaltet ist und einen Außenumfang des Stents (1) umgibt, bevor sie freigegeben wird, und sich keine der zwei Enden der Schürze (2) in der entfalteten Konfiguration in einer axialen Richtung des Stents (1) über die entsprechende Seite des Stents (1) erstreckt.

2. Stenteinrichtung mit Schürze zum Verhindern der peripheren Leckage nach Anspruch 1, wobei eine axiale Seitenkante des Stents (1) eine scharfwinklige Struktur aufweist und bevor der Stent (1) freigegeben wird, sich eine entsprechende Seite der Schürze (2) nicht über die Spitzen der scharfwinkligen Struktur axial hinaus erstreckt.

3. Stenteinrichtung mit Schürze zum Verhindern der peripheren Leckage nach Anspruch 1, wobei, bevor der Stent (1) freigegeben wird, die Schürze (2) in der entfalteten Konfiguration eine gefaltete Struktur in einer Umfangsrichtung aufweist.

4. Stenteinrichtung mit Schürze zum Verhindern der peripheren Leckage nach Anspruch 1, wobei ein Ende des Antriebsabschnitts (4a) mit einem Festpunkt (5a) des Stents verbunden ist und der Antriebsabschnitt (4a) mit dem Kraftausübungsabschnitt (4b) verbunden ist, nachdem er durch einen Wendepunkt (5b) des Stents (1) hindurch passiert ist; und eine relative Umfangsverschiebung zwischen dem Festpunkt (5a) und dem Wendepunkt (5b), nachdem der Stent (1) freigesetzt wird, im Vergleich zu derjenigen, bevor der Stent (1) freigesetzt wird, produziert wird.

5. Stenteinrichtung mit Schürze zum Verhindern der peripheren Leckage nach Anspruch 4, wobei 2 bis 8 Zugschnüre (4) bereitgestellt sind, die um den Stent (1) herum in Umfangsrichtung angeordnet sind, und der Stent (1) mit einem Führungsloch (5b) zum Definieren des Wendepunkts (5b) versehen ist.

6. Stenteinrichtung mit Schürze zum Verhindern der peripheren Leckage nach Anspruch 5, wobei das Führungsloch (5b) auf mindestens eine der folgenden Arten bereitgestellt wird: durch eine Öffnung in dem Stent (1), durch einen inhärenten Raum, der in dem Stent (1) ausgebildet wird, oder durch einen Ring, der zusätzlich bereitgestellt und an dem Stent (1) angebracht wird.

7. Stenteinrichtung mit Schürze zum Verhindern der peripheren Leckage nach Anspruch 4, wobei mindestens ein Abschnitt der Schürze in der axialen Richtung als ein schwimmender Abschnitt um den Außenumfang des Stents herum konfiguriert ist und der schwimmende Abschnitt eine gestapelte Konfiguration aufweist, die aus dem Zug der Zugschnur (4) resultiert; ein oder mehrere Einfädellöcher (5c) auf dem schwimmenden Abschnitt in einer axialen Richtung bereitgestellt sind und die Zugschnur (4) sich nach dem Passieren durch den Wendepunkt (5b) hindurch durch die ein oder mehreren Einfädellöcher (5c) hindurch in den schwimmenden Abschnitt hinein und aus diesem heraus fädelt.

8. Stenteinrichtung mit Schürze zum Verhindern der peripheren Leckage nach Anspruch 7, wobei sich jede Zugschnur (4) durch 1 bis 6 Einfädellöcher (5c) hindurch fädelt, deren Umfangspositionen miteinander ausgerichtet oder auf eine versetzte Art angeordnet sind.

9. Stenteinrichtung mit Schürze zum Verhindern der peripheren Leckage nach Anspruch 7, wobei auf einem schwimmenden Abschnitt angrenzend an den Endknoten des Stents (1) mindestens ein Einfädelloch (5c) bereitgestellt ist.

10. Stenteinrichtung mit Schürze zum Verhindern der peripheren Leckage nach Anspruch 4, wobei der Stent (1) sich in einer zylindrischen Geflechtstruktur befindet, die eine Vielzahl von Zellen aufweist, und ein Umfangsabstand zwischen dem Festpunkt (5a) und dem Wendepunkt (5b) des Stents (1), der der Zugschnur (4) entspricht, 1 bis 4 Zellen des Stents (1) umspannt.

11. Stenteinrichtung mit Schürze zum Verhindern der peripheren Leckage nach Anspruch 10, wobei die axialen Positionen des Festpunkts (5a) und des Wendepunkts (5b) aneinander ausgerichtet sind oder auf eine versetzte Art angeordnet sind.

12. Stenteinrichtung mit Schürze zum Verhindern der peripheren Leckage nach Anspruch 7, wobei die Zugschnur (4) und der schwimmende Abschnitt auf die folgende Art verbunden sind: ein Ende der Zugschnur (4), das von dem Festpunkt (5a) entfernt ist, mit einem distalen Ende eines schwimmenden Abschnitts verbunden ist, das zuerst freigegeben werden soll, und der Wendepunkt (5b) sich an einem proximalen Ende des schwimmenden Abschnitts, das später freigegeben werden soll, oder angrenzend an dieses befindet, und die axiale Position des Festpunkts (5a) weiter von dem proximalen Ende des schwimmenden Abschnitts als der Wendepunkt (5b) entfernt ist; oder ein Ende der Zugschnur (4), das von dem Festpunkt (5a) entfernt ist, mit einem proximalen Ende eines schwimmenden Abschnitts verbunden ist, das später freigegeben werden soll, und der Wendepunkt (5b) sich an einem distalen Ende schwimmenden Abschnitts, das zuerst freigegeben werden soll, oder angrenzend an dieses befindet, und die axiale Position des Festpunkts (5a) weiter von dem distalen Ende des schwimmenden Abschnitts als der Wendepunkt (5b) entfernt ist.

13. Stenteinrichtung mit Schürze zum Verhindern der peripheren Leckage nach Anspruch 1, wobei sich die Zugschnur (4) in der axialen Richtung des Stents (1) wellt, während sie sich in Umfangsrichtung entlang des Stents (1) erstreckt und so eine wellenförmige Struktur ausbildet.

14. Stenteinrichtung mit Schürze zum Verhindern der peripheren Leckage nach Anspruch 1, wobei sich die Zugschnur (4) zwischen der Schürze (2) und dem Stent (1) in Umfangsrichtung fädelt.

15. Herzklappe, umfassend eine Stenteinrichtung und Klappen, die in der Stenteinrichtung bereitgestellt sind, wobei die Stenteinrichtung die Stenteinrichtung mit Schürze zum Verhindern der peripheren Leckage nach einem der Ansprüche 1 bis 14 ist.

**Revendications**

1. Appareil formant stent avec jupe destiné à prévenir des fuites périphériques, comprenant un stent (1) qui a une première configuration avant d'être libéré et une seconde configuration après avoir été libéré qui est différente de la première configuration, l'appareil formant stent est en outre fourni avec une jupe flexible (2), dans lequel la jupe flexible (2) a :

une configuration empilée, dans laquelle la jupe (2) est pliée et empilée dans la direction axiale du stent (1) après avoir été libérée et forme un obturateur de fuite périphérique annulaire ; dans lequel au moins une partie de la jupe est fixée sur le stent, et l'appareil formant stent pour prévenir des fuites périphériques est en outre fourni avec une corde de traction (4) pour entraîner la jupe (2) à se transformer en la configuration empilée, la corde de traction (4) comprend une partie d'entraînement (4a) et une partie exerçant uneforce (4b), dans lequel la partie d'entraînement (4a) fonctionne en coopération avec le stent (1) pendant la libération, et la partie exerçant une force (4b) est reliée à la jupe (2) et est tirée par la partie d'entraînement (4a) pendant la libération du stent (1) pour obtenir un déplacement axial par rapport au stent (1) de manière à tirer la jupe (2) pour qu'elle soit empilée ; **caractérisé en ce que** la jupe flexible (2) a en outre une configuration dépliée, dans laquelle la jupe (2) se déplie axialement et entoure une périphérie externe du stent (1) avant d'être libérée, et aucune des deux extrémités de la jupe (2) dans la configuration dépliée dans une direction axiale du stent (1) ne s'étend au-delà du côté correspondant du stent (1).

2. Appareil formant stent avec jupe pour prévenir des fuites périphériques selon la revendication 1, dans lequel un bord latéral axial du stent (1) a une structure à angle aigu, et avant que le stent (1) ne soit libéré, un côté correspondant de la jupe (2) ne s'étend pas axialement au-delà des pointes de la structure à angle aigu.

3. Appareil formant stent avec jupe pour prévenir les fuites périphériques selon la revendication 1, dans lequel avant que le stent (1) ne soit libéré, la jupe (2) dans la configuration dépliée a une structure pliée dans une direction circonférentielle.

4. Appareil formant stent avec jupe pour prévenir des fuites périphériques selon la revendication 1, dans lequel une extrémité de la partie d'entraînement (4a) est reliée à un point fixe (5a) du stent, et la partie d'entraînement (4a) est reliée à la partie exerçant une force (4b) après avoir traversé un point de rotation (5b) du stent (1) ; et un déplacement circonférentiel relatif entre le point fixe (5a) et le point de rotation (5b) est produit après que le stent (1) a été libéré par rapport à ce qu'il était avant que le stent (1) ne soit libéré.

5. Appareil formant stent avec jupe pour prévenir des fuites périphériques selon la revendication 4, dans lequel 2 à 8 cordes de traction (4) sont fournies qui sont agencées circonférentiellement autour du stent (1), et le stent (1) est fourni avec un trou de guidage (5b) pour définir le point de rotation (5b).

6. Appareil formant stent avec jupe pour prévenir des fuites périphériques selon la revendication 5, dans lequel le trou de guidage (5b) est fourni par au moins une des manières suivantes : par une ouverture dans le stent (1), par un espace inhérent formé dans le stent (1), ou par un anneau supplémentaire fourni et fixé au stent (1).

7. Appareil formant stent avec jupe pour prévenir des fuites périphériques selon la revendication 4, dans lequel au moins une section de la jupe dans la direction axiale est conçue comme une section flottante autour de la périphérie externe du stent, et la section flottante a une configuration empilée résultant de la traction de la corde de traction (4) ; un ou plusieurs trous d'enfilage (5c) sont fournis sur la section flottante dans une direction axiale, et la corde de traction (4) s'enfile dans ou se retire de la section flottante à travers le ou les trous d'enfilage (5c) après avoir traversé le point de rotation (5b).

8. Appareil formant stent avec jupe pour prévenir des fuites périphériques selon la revendication 7, dans lequel chaque corde de traction (4) s'enfile à travers 1 à 6 trous d'enfilage (5c), dont les positions circonférentielles sont alignées les unes avec les autres ou agencées en quinconce.

9. Appareil formant stent avec jupe pour prévenir des fuites périphériques selon la revendication 7, dans lequel au moins un trou d'enfilage (5c) est fourni sur une section flottante adjacente aux noeuds d'extrémité du stent (1).

10. Appareil formant stent avec jupe pour prévenir des fuites périphériques selon la revendication 4, dans lequel le stent (1) est dans une structure cylindrique maillée ayant une pluralité de cellules, et une distance circonférentielle entre le point fixe (5a) et le point de rotation (5b) du stent (1) correspondant à la corde de traction (4) s'étend sur 1 à 4 cellules du stent (1).

11. Appareil formant stent avec jupe pour prévenir des fuites périphériques selon la revendication 10, dans lequel les positions axiales du point fixe (5a) et du point de rotation (5b) sont alignées l'une sur l'autre ou agencées en quinconce.

12. Appareil formant stent avec jupe pour prévenir des fuites périphériques selon la revendication 7, dans lequel la corde de traction (4) et la section flottante sont reliées de la manière suivante : une extrémité de la corde de traction (4) éloignée du point fixe (5a) est reliée à une extrémité distale d'une section flottante qui doit être libérée en premier, et le point de rotation (5b) est situé ou adjacent à une extrémité proximale de la section flottante qui doit être libérée plus tard, et la position axiale du point fixe (5a) est plus éloignée de l'extrémité proximale de la section flottante que le point de rotation (5b) ; ou une extrémité de la corde de traction (4) éloignée du point fixe (5a) est reliée à une extrémité proximale d'une section flottante devant être libérée ultérieurement, et le point de rotation (5b) est situé ou adjacent à une extrémité distale de la section flottante devant être libérée en premier, et la position axiale du point fixe (5a) est plus éloignée de l'extrémité distale de la section flottante que le point de rotation (5b).

13. Appareil formant stent avec jupe pour prévenir des fuites périphériques selon la revendication 1, dans lequel la corde de traction (4) ondule dans la direction axiale du stent (1) tout en s'étendant le long du stent (1) dans une direction circonférentielle et forme ainsi une structure en forme de vague.

14. Appareil formant stent avec jupe pour prévenir des fuites périphériques selon la revendication 1, dans lequel la corde de traction (4) s'enfile circonférentiellement entre la jupe (2) et le stent (1).

15. Valvule cardiaque comprenant un appareil formant stent et des valvules fournies dans l'appareil formant stent, dans laquelle l'appareil formant stent est l'appareil formant stent avec jupe pour prévenir des fuites périphériques selon l'une quelconque des revendications 1 à 14.

FIG. 1a

FIG. 2a

FIG. 2b

FIG. 2c

FIG. 2d

FIG. 2e

FIG. 2f

FIG. 2g

FIG. 2h

FIG. 3

FIG. 4

FIG. 5a

FIG. 5b

FIG. 6

FIG. 7a

FIG. 7b

FIG. 7c

FIG. 7d

FIG. 7e

FIG. 8

FIG. 9

FIG. 10a

FIG. 10b

FIG. 10c

FIG. 11

FIG. 12

FIG. 13

FIG. 14a

FIG. 14b

FIG. 14c

X6　　X7　　X8

Y5　　　　Y6

FIG. 15

FIG. 16a

X9

A

4

2

Y7

FIG. 16b

2h

2i

1

2j

FIG. 16c

2h

2i

2j

FIG. 16d

4

2

FIG. 17

4

2

FIG. 18

4

2

FIG. 19a

FIG. 19b

FIG. 19c

43

EP 3 679 891 B1

FIG. 20

FIG. 21

FIG. 22

FIG. 23

FIG. 24

FIG. 25

FIG. 26

FIG. 27

FIG. 28a

FIG. 28b

FIG. 29

FIG. 30

FIG. 31

FIG. 32a

FIG. 32b

FIG. 32c

FIG. 33a

FIG. 33b

FIG. 33c

**EP 3 679 891 B1**

FIG. 34a

FIG. 34b

FIG. 34c

51

FIG. 34d

FIG. 34e

FIG. 34f

FIG. 34g

**EP 3 679 891 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009044082 A2